# EUROPEAN PATENT APPLICATION

(11) **EP 3 674 709 A1**
(43) Date of publication of application: **01.07.2020**
(21) Application number: 19211759.6
(22) Date of filing: 07.11.2016
(51) Int. Cl.: G01N 33/574

(54) **FLOW CYTOMETRY FOR MONITORING HISTONE H3 METHYLATION STATUS**

(30) Priority: 05.11.2015 US 201562251322 P; 17.06.2016 US 201662351877 P
(62) Divisional of application: 16810124.4
(71) Applicant: Epizyme Inc, Cambridge, MA 02139 (US)
(72) Inventor: PLESCIA, Christopher, Cambridge, MA 02139 (US)
(74) Representative: Russell, Tim

(57) **Abstract**

The present disclosure relates to methods of detecting histone epigenetic modifications and compositions comprising inhibitors of human histone methyltransferase EZH2 and their use for the treatment of cancer.

## Description

### Related Applications

This application claims priority to, and the benefit of U.S. Provisional Application Nos. 62/251,322 filed November 5, 2015, and 62/351,877 filed June 17, 2016, the contents of each of which are incorporated herein by reference in their entireties.

### BACKGROUND

Modification of histones through, for example, methylation by histone methyltransferases (HMTs), is associated with changes in gene expression. Specifically, HMTs regulate gene expression through the placement of one or more methyl groups (methyl marks) onto specific lysine and arginine residues of histone proteins. Genetic alterations of histone protein sequences may alter HMT activity and, as a consequence, misregulate gene expression and result in aberrant placement of methyl marks onto histones.

Mutations in EZH2 and/or overactivity of this histone methyltransferase have been associated with various kinds of cancers. There is an unmet need for an effective EZH2 inhibitor for use in anticancer treatment. Moreover, there is an unmet need for methods to assess epigenetic modifications in a subject.

### SUMMARY

Some aspects of this disclosure provide methods, strategies, and assays for determining epigenetic modifications in cells or tissues of interest. Some aspects of this disclosure provide methods, strategies, and assays for detecting, determining, and/or monitoring epigenetic modifications that are associated with a disease, *e.g.,* with cancer, in a subject. Some aspects of this disclosure provide methods, strategies, and assays for the detection of disease states by measuring the epigenetic status in cells and tissues of interest. Also provided herein are methods and assays for measuring epigenetic modifications, *e.g.,* histone methylation, in a biological sample, *e.g.,* by flow cytometry. The methods, strategies, and assays provided herein are useful, *e.g.,* for the detection of a disease state in a subject, tissue, or cell; for determining whether a cell or tissue associated with a disease or disorder, *e.g.,* a cancer cell or a tumor in a subject, is sensitive to treatment with a therapeutic agent modulating an epigenetic modification associated with the disease or disorder, *e.g.,* to treatment with an EZH2 inhibitor; and for monitoring the effect of treatment of a target cell or tissue, *e.g.,* of a tumor in a subject, with an epigenetic modulator, *e.g.,* with an EZH2 inhibitor.

In some embodiments, the disclosure provides a flow-cytometry method comprising quantifying trimethylation of lysine 27 of histone H3, quantifying total H3 level, and immunophenotyping of discrete cell populations. In some embodiments of this method, the discrete cell populations comprise a population of cells obtained from a subject.

In some embodiments of flow cytometry methods of the disclosure, the population of cells comprises, consists essentially, or consists of lymphoid cells. In some embodiments, the population of cells comprises, consists essentially, or consists of myeloid cells. In some embodiments, the population of cells comprises, consists essentially, or consists of myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells, or any combination thereof. In some embodiments, the population of cells is isolated from peripheral blood or from a population of peripheral blood mononuclear cells obtained from the subject.

In some embodiments of flow cytometry methods of the disclosure, the population of cells is characterized by expression of a cell surface antigen or a combination of cell surface antigens. In some embodiments, the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof. In some embodiments, the cell surface antigen is CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR, or any combination thereof.

In some embodiments of flow cytometry methods of the disclosure, the population of cells comprises, essentially consists of, or consists of myeloid stem or progenitor cells that are CD133⁺, CD271⁺, CD11⁺, and/or CD347⁺; erythroblasts that are CD71⁺; megakaryocytes that are CD61⁺; platelets that are CD61⁺; granulocytes that are HLA-DR⁺ and CD14⁻; basophils that are CD123⁺; eosinophils that are CD44⁺; neutrophils that are CD15⁺ and/or CD16⁺; monocytes that are CD14⁺, CD11b⁺, CD16⁻, and/or HLA-DR⁺; MDClc cells that are CD1c⁺; MDC2 cells that are CD141⁺; mast cells that are CD117⁺; lymphoid stem or progenitor cells that are CD34⁺, CD133⁺, CD271⁺, CD117⁺; T-lymphocytes (T-cells) that are CD2⁺ and/or CD3⁺; cytotoxic T cells that are CD8⁺; T helper cell that CD4⁺; regulatory T-cells that are CD4⁺ and CD25⁺; NK/T cell that are CD3⁺ and CD56⁺; natural killer T cells that are CD56⁺; activated T cells that are CD25⁺ and CD30⁺; B-cells that are CD19⁺ and/or CD20⁺; activated B-cells that are CD19⁺, CD25⁺, and/or CD30⁺; plasma cells that are CD138⁺; natural killer B cells that are CD56⁺; natural killer (NK) cells that are CD3⁻, CD19⁻, HLA-DR⁺ and CD16⁻; plasmacytoid dendritic cells that are CD304⁺; or any combination thereof.

In some embodiments of flow cytometry methods of the disclosure, the population of cells comprises, essentially consists of, or consists of B-cells.

In some embodiments of flow cytometry methods of the disclosure, the population of cells comprises, essentially consists of, or consists of T cells.

In some embodiments of flow cytometry methods of the disclosure, the population of cells comprises, essentially consists of, or consists of monocytes. In some embodiments, the monocytes are CD14⁺ and CD16⁻; CD14^{low} and CD16⁺; or CD14^{high} and CD16^{low} monocytes, or any combination thereof.

In some embodiments of flow cytometry methods of the disclosure, the population of cells comprises, essentially consists of, or consists of granulocytes. In some embodiments, the granulocytes are neutrophils, eosinophils, or mast cells, or any combination thereof.

In some embodiments of flow cytometry methods of the disclosure, quantifying trimethylation of lysine 27 of histone 3 comprises measuring a level of trimethylated lysine 27 in histone 3 in the population of cells, measuring the total level of histone 3 in the population of cells, and calculating a ratio of trimethylated lysine 27 level in histone 3 to the total histone 3 level in the population of cells.

In some embodiments of flow cytometry methods of the disclosure, the method further comprises obtaining a blood sample or a sample of blood cells from the subject. In some embodiments, the method further comprises isolating the population of cells. In some embodiments, the isolating is by flow cytometry. In some embodiments, the flow cytometry is based on the expression of one or more cell surface antigens. In some embodiments, the isolating is by fluorescence-activated cell sorting (FACS). In some embodiments, the isolating is by magnetic-activated cell sorting (MACS).

In some embodiments of flow cytometry methods of the disclosure, the subject (a) has been or is scheduled to be administered a therapeutically-effective amount of an EZH2 inhibitor, or (b) is identified as having increased H3K27me3 amounts in comparison to a control or reference value.

In some embodiments of flow cytometry methods of the disclosure, the quantifying trimethylation of lysine 27 of histone H3 is performed before and after the subject is administered a therapeutically-effective amount of an EZH2 inhibitor.

In some embodiments of flow cytometry methods of the disclosure, the method is used to monitor trimethylation of lysine 27 of histone H3 in a subject during a time period in which a therapeutically-effective amount of an EZH2 inhibitor is administered to the subject.

In some embodiments, the disclosure provides a method, comprising, quantifying trimethylation of lysine 27 of Histone H3 in a population of cells obtained from a subject. In some embodiments of this method,

In some embodiments of a method comprising quantifying trimethylation of lysine 27 of Histone H3 in a population of cells obtained from a subject, the population of cells comprises, consists essentially, or consists of lymphoid cells. In some embodiments, the population of cells comprises, consists essentially, or consists of myeloid cells. In some embodiments, the population of cells comprises, consists essentially, or consists of myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells, or any combination thereof. In some embodiments, the population of cells is isolated from peripheral blood or from a population of peripheral blood mononuclear cells obtained from the subject.

In some embodiments of a method comprising quantifying trimethylation of lysine 27 of Histone H3 in a population of cells obtained from a subject, the population of cells is characterized by expression of a cell surface antigen or a combination of cell surface antigens. In some embodiments, the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof. In some embodiments, the cell surface antigen is CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR, or any combination thereof.

In some embodiments of a method comprising quantifying trimethylation of lysine 27 of Histone H3 in a population of cells obtained from a subject, the population of cells comprises, essentially consists of, or consists of myeloid stem or progenitor cells that are CD133⁺, CD271⁺, CD11⁺, and/or CD347⁺; erythroblasts that are CD71⁺; megakaryocytes that are CD61⁺; platelets that are CD61⁺; granulocytes that are HLA-DR⁺ and CD14⁻; basophils that are CD123⁺; eosinophils that are CD44⁺; neutrophils that are CD15⁺ and/or CD16⁺; monocytes that are CD14⁺, CD11b⁺, CD16⁻, and/or HLA-DR⁺; MDClc cells that are CD1c⁺; MDC2 cells that are CD141⁺; mast cells that are CD117⁺; lymphoid stem or progenitor cells that are CD34⁺, CD133⁺, CD271⁺, CD117⁺; T-lymphocytes (T-cells) that are CD2⁺ and/or CD3⁺; cytotoxic T cells that are CD8⁺; T helper cell that CD4⁺; regulatory T-cells that are CD4⁺ and CD25⁺; NK/T cell that are CD3⁺ and CD56⁺; natural killer T cells that are CD56⁺; activated T cells that are CD25⁺ and CD30⁺; B-cells that are CD19⁺ and/or CD20⁺; activated B-cells that are CD19⁺, CD25⁺, and/or CD30⁺; plasma cells that are CD138⁺; natural killer B cells that are CD56⁺; natural killer (NK) cells that are CD3⁻, CD19⁻, HLA-DR⁺ and CD16⁻; plasmacytoid dendritic cells that are CD304⁺; or any combination thereof.

In some embodiments of a method comprising quantifying trimethylation of lysine 27 of Histone H3 in a population of cells obtained from a subject, the population of cells comprises, essentially consists of, or consists of B-cells.

In some embodiments of a method comprising quantifying trimethylation of lysine 27 of Histone H3 in a population of cells obtained from a subject, the population of cells comprises, essentially consists of, or consists of T cells.

In some embodiments of a method comprising quantifying trimethylation of lysine 27 of Histone H3 in a population of cells obtained from a subject, the population of cells comprises, essentially consists of, or consists of monocytes. In some embodiments, the monocytes are CD14⁺ and CD16⁻; CD14^{low} and CD16⁺; or CD14^{high} and CD16^{low} monocytes, or any combination thereof.

In some embodiments of a method comprising quantifying trimethylation of lysine 27 of Histone H3 in a population of cells obtained from a subject, the population of cells comprises, essentially consists of, or consists of granulocytes. In some embodiments, the granulocytes are neutrophils, eosinophils, or mast cells, or any combination thereof.

In some embodiments of a method comprising quantifying trimethylation of lysine 27 of Histone H3 in a population of cells obtained from a subject, quantifying trimethylation of lysine 27 of histone 3 comprises measuring a level of trimethylated lysine 27 in histone 3 in the population of cells, measuring the total level of histone 3 in the population of cells, and calculating a ratio of trimethylated lysine 27 level in histone 3 to the total histone 3 level in the population of cells.

In some embodiments of a method comprising quantifying trimethylation of lysine 27 of Histone H3 in a population of cells obtained from a subject, the method further comprises obtaining a blood sample or a sample of blood cells from the subject. In some embodiments, the method further comprises isolating the population of cells. In some embodiments, the isolating is by flow cytometry. In some embodiments, the flow cytometry is based on the expression of one or more cell surface antigens. In some embodiments, the isolating is by fluorescence-activated cell sorting (FACS). In some embodiments, the isolating is by magnetic-activated cell sorting (MACS).

In some embodiments of a method comprising quantifying trimethylation of lysine 27 of Histone H3 in a population of cells obtained from a subject, the subject (a) has been or is scheduled to be administered a therapeutically-effective amount of an EZH2 inhibitor, or (b) is identified as having increased H3K27me3 amounts in comparison to a control or reference value.

In some embodiments of a method comprising quantifying trimethylation of lysine 27 of Histone H3 in a population of cells obtained from a subject, the quantifying trimethylation of lysine 27 of histone H3 is performed before and after the subject is administered a therapeutically-effective amount of an EZH2 inhibitor.

In some embodiments of a method comprising quantifying trimethylation of lysine 27 of Histone H3 in a population of cells obtained from a subject, the method is used to monitor trimethylation of lysine 27 of histone H3 in a subject during a time period in which a therapeutically-effective amount of an EZH2 inhibitor is administered to the subject.

The disclosure provides a method of quantifying an epigenetic modification of a histone in a sample from a subject, comprising (a) contacting at least one cell in the sample with a permeabilizing composition to obtain a permeabilized sample; (b) contacting the permeabilized sample with a first fluorescent-conjugated antibody that specifically binds an epitope within H3K27me1, H3K27me2, or H3K27me3; (c) contacting the permeabilized sample with a second fluorescent-conjugated antibody that specifically binds an epitope within H3; (d) detecting a first fluorescent signal from the first fluorescent-conjugated antibody; (e) detecting a second fluorescent signal from the second-fluorescent conjugated antibody; and (d) determining an amount of H3 that is epigenetically-modified comprising dividing a value of the first fluorescent signal by a value of the second fluorescent signal, thereby quantifying the epigenetic modification of the histone in the sample.

Methods of quantifying an epigenetic modification of a histone may further comprise sorting the at least one cell in the sample by cell size prior to contacting the permeabilized sample with either the first fluorescent-conjugated antibody or the second fluorescent-conjugated antibody.

Methods of quantifying an epigenetic modification of a histone may further comprise immunophenotyping the at least one cell in the sample, comprising contacting the sample with a third fluorescent-conjugated antibody that specifically binds a third epitope on a cell type specific marker, and detecting a third fluorescent signal from the third fluorescent-conjugated antibody. In certain embodiments of the methods of the disclosure, the cell type specific marker is a cell surface antigen. In some embodiments, the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof. In some embodiments, the cell surface antigen may be selected from the group consisting of CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR.

The disclosure provides a method of monitoring a status of an epigenetic modification of a histone in a sample from a subject in need thereof comprising, (a) determining a first quantity of an epigenetic modification of a histone according to any method provided above prior to an event; (b) determining a second quantity of the epigenetic modification of the histone according to any method provided above; and (c) comparing the first quantity to the second quantity, wherein an increase indicates increased epigenetic modification following the event and a decrease indicates decreased epigenetic modification following the event. In certain embodiments, the event is a treatment comprising administration of an EZH2 inhibitor to the subject. The subject may have or may be diagnosed with cancer. The epigenetic modification may be methylation of lysine 27 of histone 3.

The disclosure provides a method for treating cancer in a subject in need thereof, comprising administering a therapeutically-effective amount of an EZH2 inhibitor to the subject wherein the subject is identified as having increased H3K27me3 amounts in comparison to the amount of H3 according to any of the above methods.

Permeabilizing compositions of the methods may comprise, consists of, or consists essentially of a reagent selected from the group consisting of Triton X-100 (polyethylene glycol p(1,1,3,3-tetramethylbutyl)-phenyl ether), Nonidet P-40 (octylphenoxypolyethoxyethanol), Tween-20, saponin, digitonin, and n-octyl-β-D-glucopyranoside. In one embodiment, the permeabilizing composition is Triton X-100 (polyethylene glycol p(1,1,3,3-tetramethylbutyl)-phenyl ether). In certain embodiments the permeabilizing composition is about 0.25% and about 5% wt/vol Triton X-100 (polyethylene glycol p(1,1,3,3-tetramethylbutyl)-phenyl ether).

In certain embodiment of the methods of the disclosure, the first fluorescent signal is detected by flow cytometry. In certain embodiments of the methods of the disclosure, the second fluorescent signal is detected by flow cytometry. In certain embodiments of the methods of the disclosure, the third fluorescent signal is detected by flow cytometry. In certain embodiments of the methods, the first, second, and third fluorescent signals may be simultaneously detected by flow cytometry. In certain embodiment of the methods, 1, 2, 3, 4, 5, 6, 7, 8, 9, and 10 fluorescent signals can be simultaneously detected by flow cytometry.

According to the methods of the disclosure the sample may comprise, consist of, or consist essentially of blood, skin, lymph fluid, lymph tissue, bone marrow, a hematological tumor, a liquid tumor, and a solid tumor. In one aspect, the blood sample comprises, consists of, or consists essentially of erythrocytes, lymphocytes, B cells, monocytes, eosinophils, basophils, neutrophils, thrombocytes, or a combination thereof. The sample may comprise, consist of, or consist essentially of B cells.

According to the methods of the disclosure the sample may comprise, consist of, or consist essentially of endothelial cells, smooth muscle cells, pericytes, blood, lymph fluid or a combination thereof. A bone marrow sample may comprise, consist of, or consist essentially of hematopoietic stem cells, stromal stem cells, myeloid tissue, yellow marrow, erythrocytes, leukocytes, or a combination thereof.

Methods of the disclosure may further comprise contacting the at least one cell in the sample with a fixing composition to obtain a fixed sample prior to contacting the fixed sample with the first fluorescently-conjugated antibody or the second fluorescently-conjugated antibody. A fixing composition of the methods of the disclosure may be a methanol-free composition. The fixing composition may be a methanol-free composition comprising formaldehyde at a concentration of between about 1% and about 10%. A methanol-free fixing composition may comprise formaldehyde at a concentration of about 4%.

In certain embodiments of the methods of the disclosure, the EZH2 inhibitor is 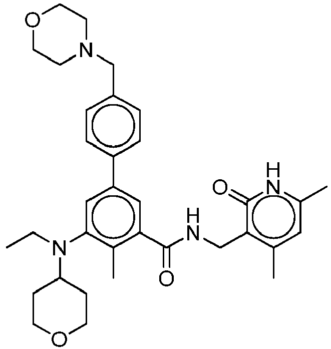 or a pharmaceutically acceptable salt thereof.

Subjects of the methods of the disclosure may be human. Human subjects may be 18 years old or younger. Subjects of the methods of the disclosure may have cancer. In certain embodiments of the methods of the disclosure, the cancer is lymphoma. Exemplary lymphomas may include, but are not limited to, diffuse large B-cell lymphoma (DLBCL), a germinal center-derived lymphoma, a non-germinal center-derived lymphoma, follicular lymphoma (FL), primary mediastinal large B-cell lymphoma (PMBCL), marginal zone lymphoma (MZL), Burkitt's lymphoma and other non-Hodgkin's lymphoma subtype.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

Other systems, processes, and features will become apparent to those skilled in the art upon examination of the following drawings and detailed description. It is intended that all such additional systems, processes, and features be included within this description, be within the scope of the present disclosure, and be protected by the accompanying claims.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The skilled artisan will understand that the drawings primarily are for illustrative purposes and are not intended to limit the scope of the inventive subject matter described herein. The drawings are not necessarily to scale; in some instances, various aspects of the inventive subject matter disclosed herein may be shown exaggerated or enlarged in the drawings to facilitate an understanding of different features. In the drawings, like reference characters generally refer to like features (*e.g.,* functionally similar and/or structurally similar elements).

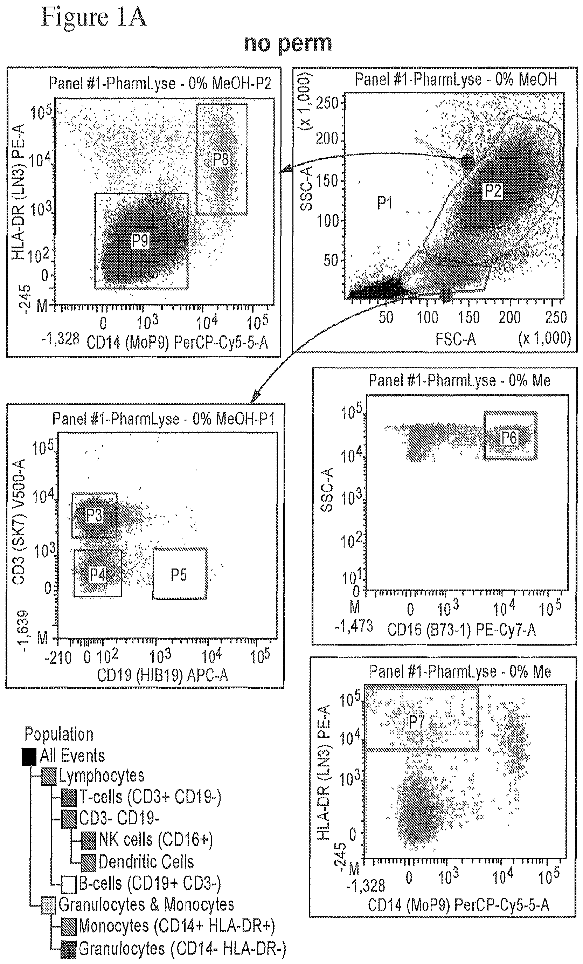

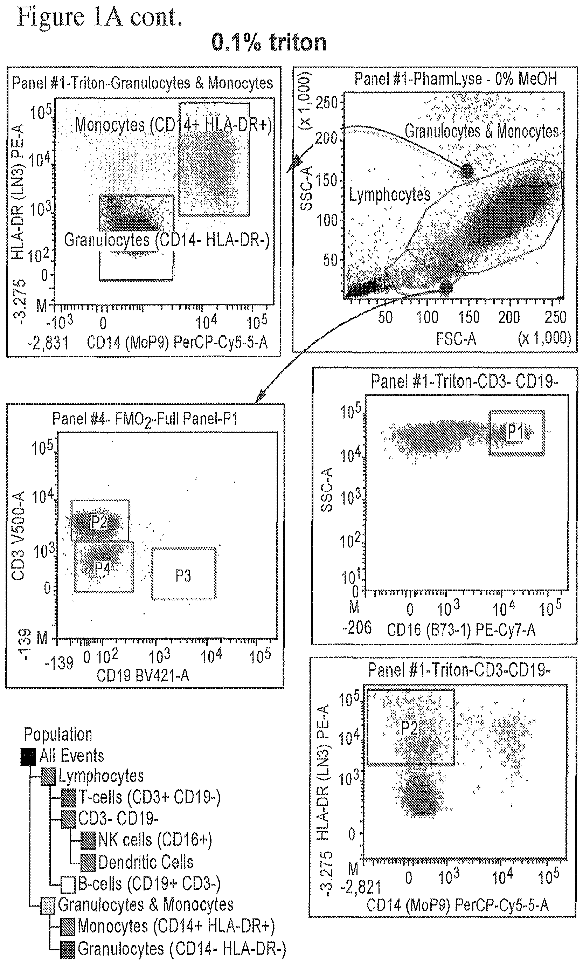

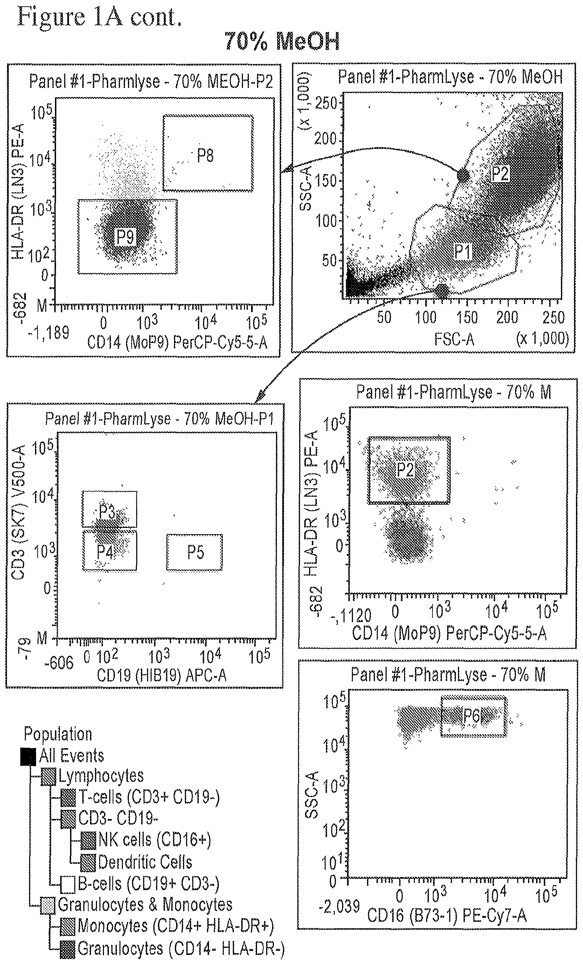

**Figure 1A** is a series of flow cytometry plots that depict a comparison of different permeabilization conditions following contact with a blood sample. The series of flow cytometry plots depicts the effect of various treatment conditions (*i.e.* no permeabilization, 0.1% Triton, 70% methanol) on the detection of blood cell derived surface epitopes including CD3, CD19, CD14, CD16 and HLA-DR (LN3). The following color code applies for all conditions: all events are colored black; all lymphocytes are colored red; T-cells (CD3+ CD19-) are colored dark blue; CD3- CD19- lymphocytes are colored purple; NK cells are colored dark green; Dendritic cells are colored light blue; B-cells (CD19+CD3-) are colored yellow; Granulocytes and monocytes are colored light green; monocytes (CD14+HLA-DR+) are colored orange; and granulocytes (CD14- HLA-DR-) are colored maroon.

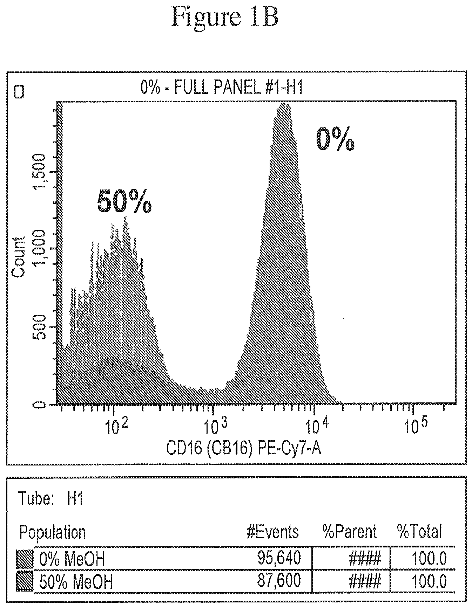

**Figure 1B** is a flow cytometry plot that depicts a comparison of 50% methanol treatment in comparison to a methanol free treatment condition on the detection of CD16 surface epitope from a blood cell sample.

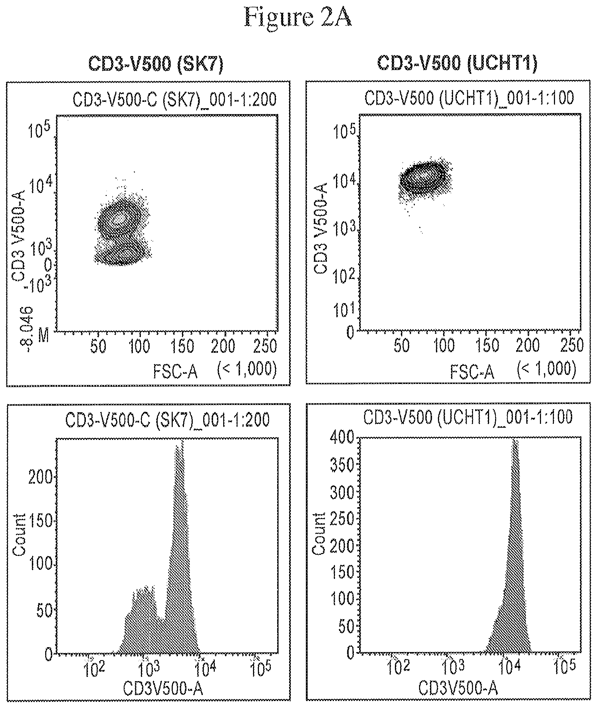

**Figure 2A** is a series of flow cytometry plots that depict binding of different clones of the CD3 antibody to a mixed population of blood cells.

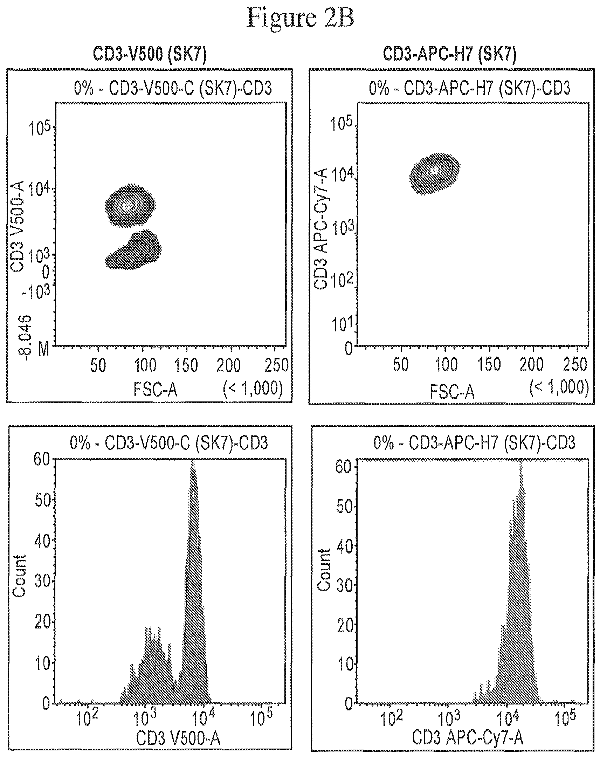

**Figure 2B** is a series of flow cytometry plots that show a comparison of the fluorophore conjugate selection (*i.e.* CD3-V500 compared with CD3-APC-H7) on the resultant fluorescent signal obtained following contact with a blood cell sample.

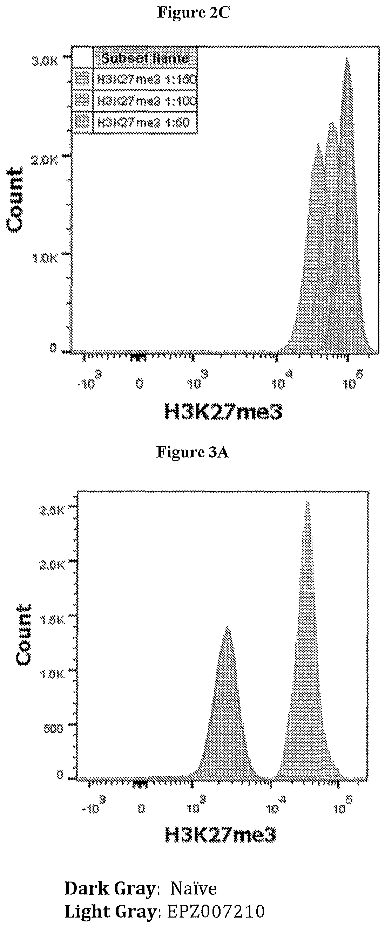

**Figure 2C** is a flow cytometry plot that shows the effect of H3K27me3 antibody titration (*i.e.* 1:50, 1:100, 1:150) on the resultant flow cytometry signal obtained following contact with a blood cell sample. The 1:150 condition (orange) is represented by the left-hand peak in this graph. The 1:100 condition (blue) is represented by the center peak in this graph. The 1:50 condition (red) is represented by the right peak in this graph.
**Figure 3A** is a flow cytometry plot that depicts H3K27me3 methylation status of OCI-LY19 cells following contact with EPZ007210 (also referred to as Compound D herein), an EZH2 inhibitor, in comparison to a no treatment condition. EPZ007210 condition represented by the left (light grey or red) peak while the native condition is represented by the right (dark grey or blue) peak.

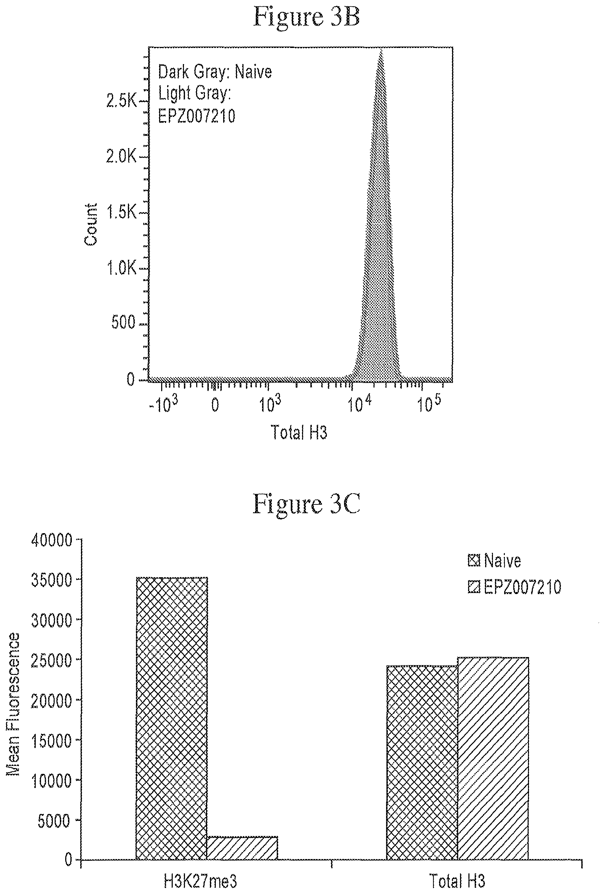

**Figure 3B** is a flow cytometry plot that depicts the detection of histone H3 in the OCI-LY19 cell line.
**Figure 3C** is a bar graph that depicts mean fluorescence as detected by flow cytometry following contact with a H3K27me3 antibody in OCI-LY19 cells that were either treated with EPZ007210 or had no treatment. Also depicted in Figure 3C is the mean fluorescence as detected by flow cytometry following contact with the total histone H3 antibody in OCI-LY19 cells following treatment with EPZ007210 or a no treatment condition.

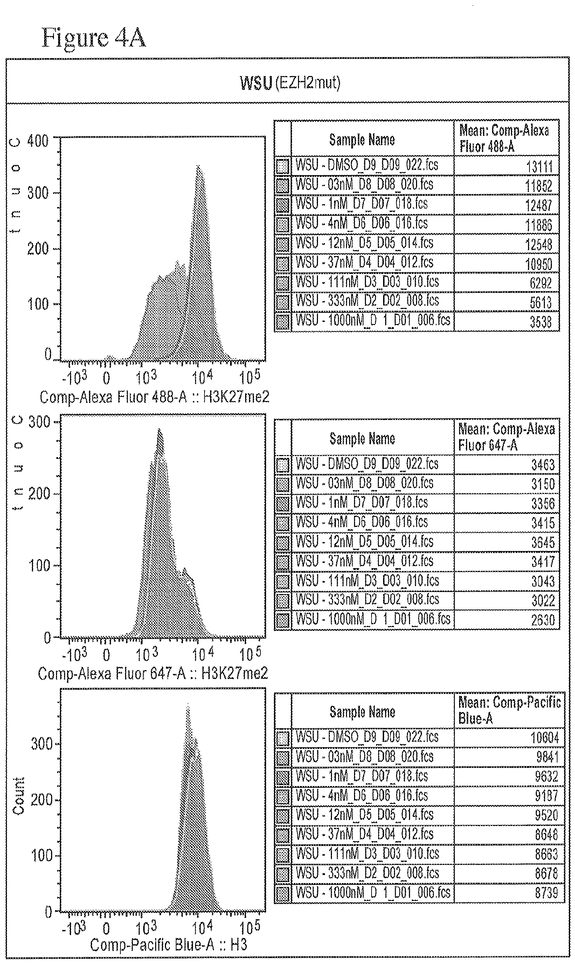

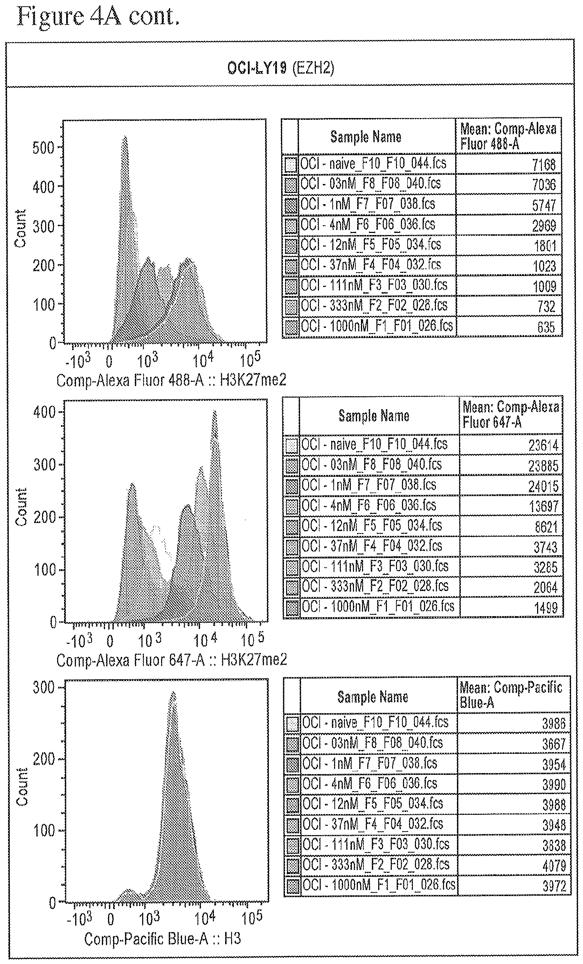

**Figure 4A** is a series of flow cytometry plots that depict the effect of incubating the WSU cell line and the OCI-LY19 cell line with various concentrations of EPZ007210 on the H3K27 methylation status of the cells. H3K27me3 status is depicted on the flow cytometry plots following incubation with the following EPZ007210 concentrations DMSO only (yellow), 0.3nM (light blue), 1nM (magenta), 4nM (pink), 12nM (dark green), 37nM (light green), 111nM (orange), 333nM (teal) and 1000nM (red).

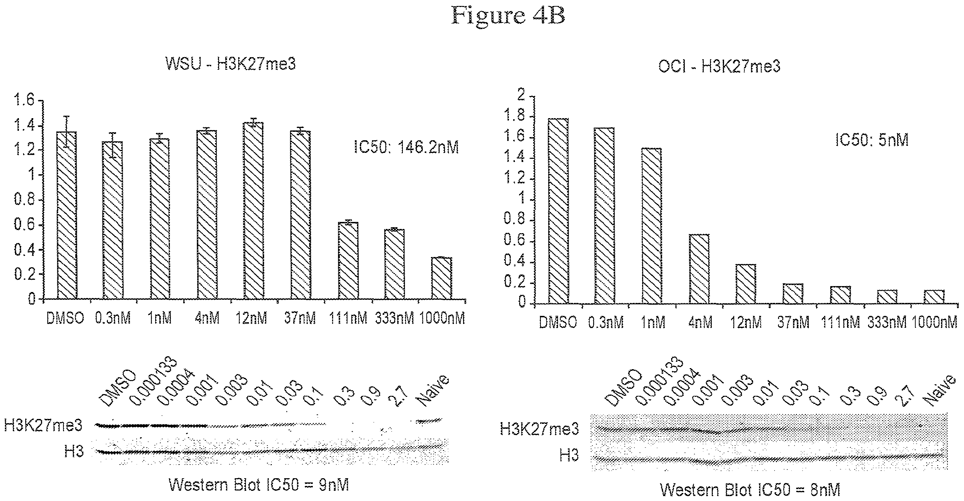

**Figure 4B** is a pair of Western blot analyses (and their corresponding graphs quantifying the results of the blot) that depict the results of a dose response study that utilized EPZ007210 to contact with the WSU cell line and the OCI-LY19 cell line. The IC₅₀ is indicated as an inset. Chromatin flow cytometry and histone Western were found to agree for OCI-LY19 cells, but diverged for WSU cells, possibly due to greater intrinsic auto-fluorescence of WSU cells.

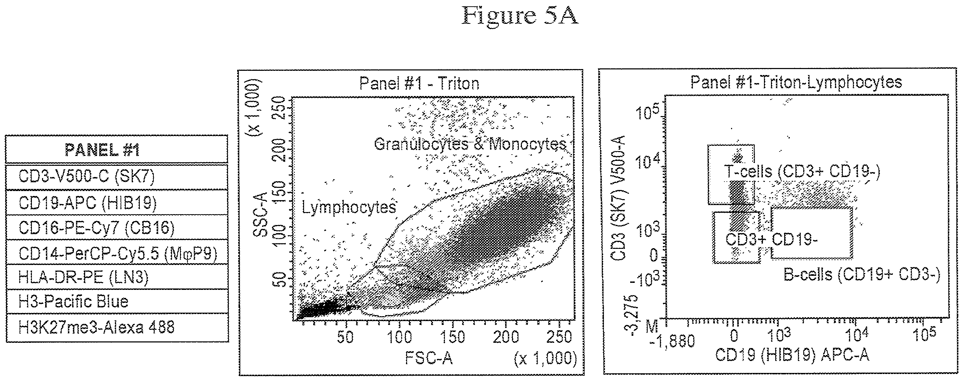

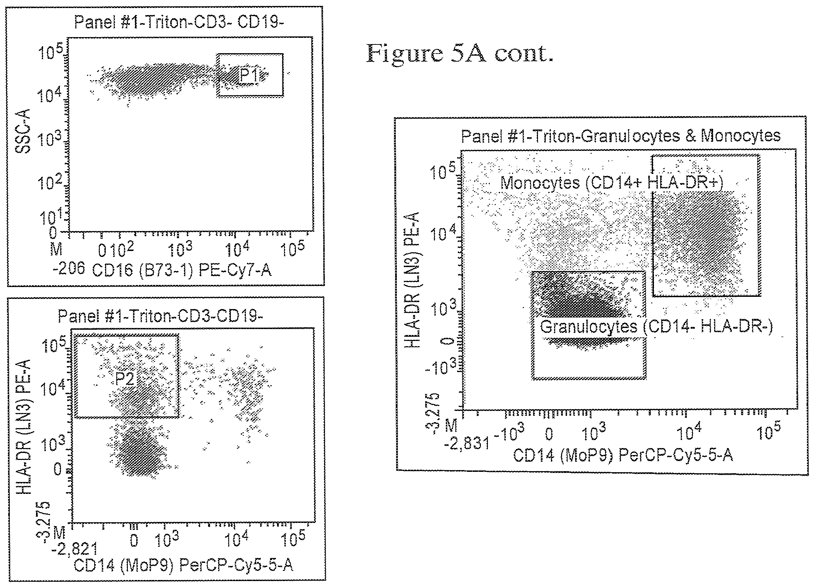

**Figure 5A** is a series of flow cytometry plots that depict the simultaneous detection by flow cytometry of cellular surface epitopes and nuclear epitopes in a blood cell sample using a panel of antibodies. The panel of antibodies represented on the flow cytometry plots are CD3-V500-C (SK7), CD19-APC (HIB19), CD16-PE-Cy7 (CD16), CD14-PerCP-Cy5.5 (M_{Ψ}P9), HLA-DR-PE (LN3), H3-Pacific Blue, and H3K27me3-Alexa-488. The following color code applies: all events are colored black; all lymphocytes are colored red; T-cells (CD3+ CD19-) are colored dark blue; CD3- CD19- lymphocytes are colored purple; NK cells are colored dark green; Dendritic cells are colored light blue; B-cells (CD19+CD3-) are colored yellow; Granulocytes and monocytes are colored light green; monocytes (CD14+HLA-DR+) are colored orange; and granulocytes (CD14-HLA-DR-) are colored maroon.

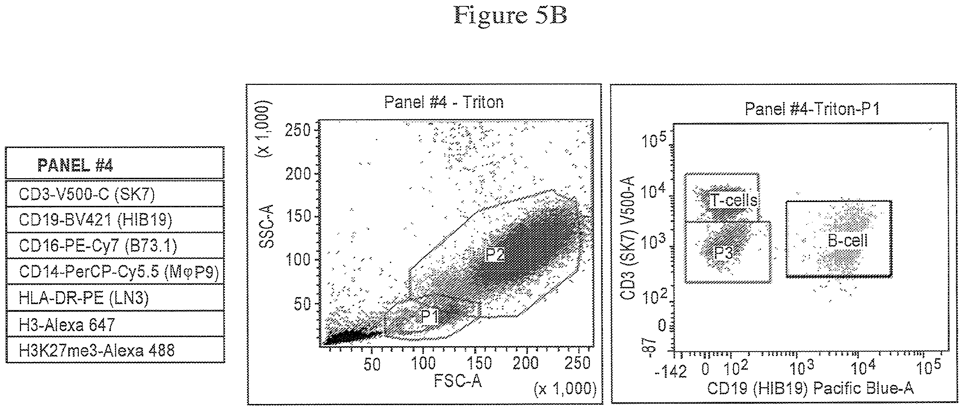

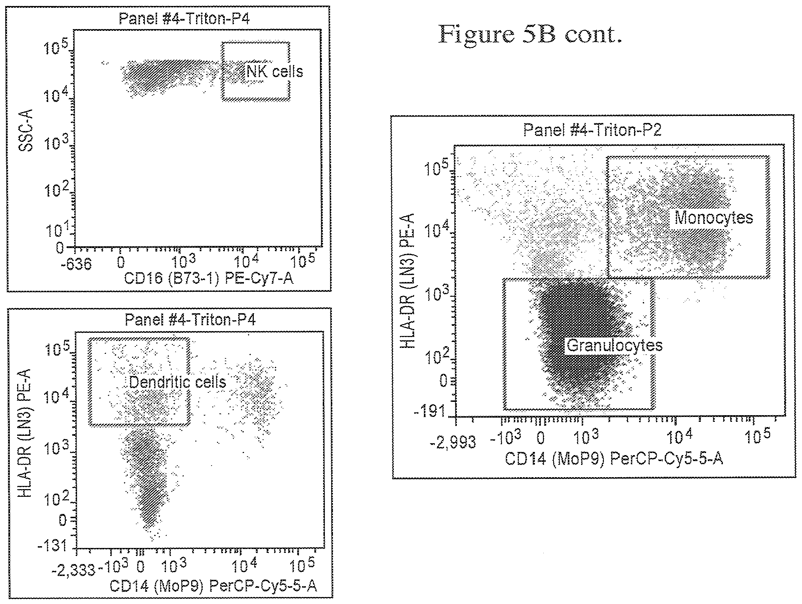

**Figure 5B** is a series flow cytometry plots that depict the simultaneous detection by flow cytometry of cellular surface epitopes and nuclear epitopes in a blood cell sample using a panel of antibodies. The panel of antibodies represented on the flow cytometry plots are CD3-V500-C (SK7), CD19-BV421 (HIB19), CD16-PE-Cy7 (B73.1), CD14-PerCP-Cy5.5 (M_{Ψ}P9), HLA-DR-PE (LN3), H3-Alexa 647, and H3K27me3-Alexa-488. The following color code applies: all events are colored black; all lymphocytes are colored red; T-cells (CD3+ CD19-) are colored dark blue; CD3- CD19- lymphocytes are colored purple; NK cells are colored dark green; Dendritic cells are colored light blue; B-cells (CD 19+CD3-) are colored yellow; Granulocytes and monocytes are colored light green; monocytes (CD14+HLA-DR+) are colored orange; and granulocytes (CD14-HLA-DR-) are colored maroon.

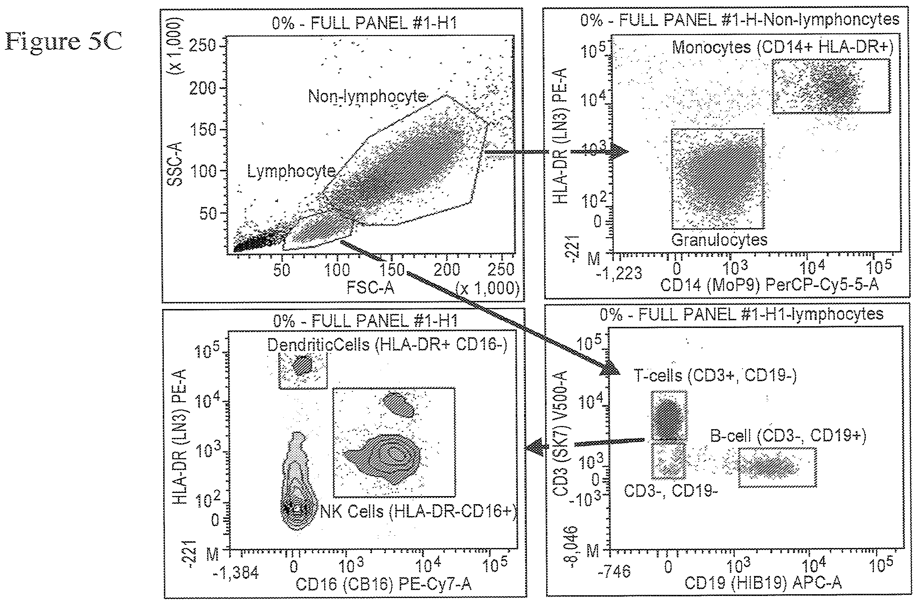

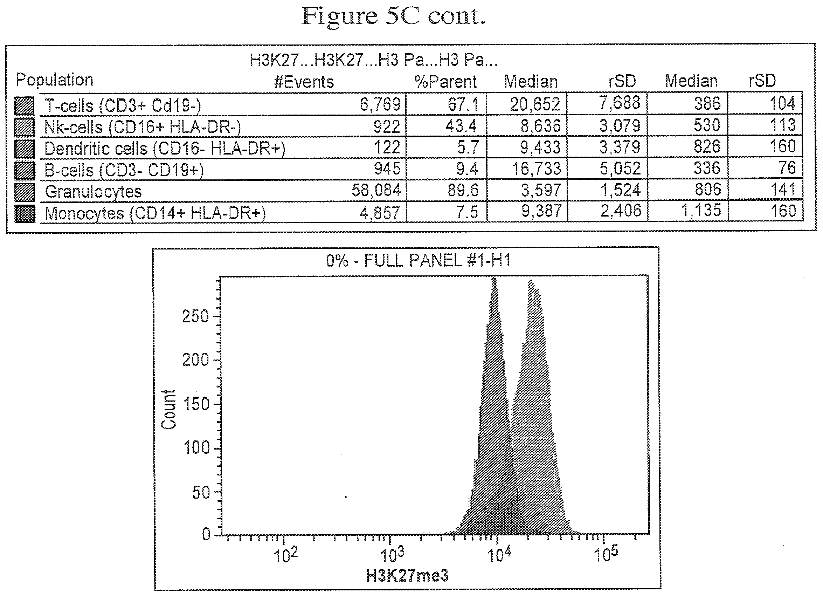

**Figure 5C** is a series of flow cytometry plots that depict the simultaneous detection by flow cytometry of cellular surface epitopes and nuclear epitopes in a blood cell sample using a panel of antibodies. The panel of antibodies represented on the flow cytometry plots is CD3-V500-C (SK7), CD19-APC (HIB19), CD16-PE-Cy7 (CD16), CD14-PerCP-Cy5.5 (M_{Ψ}P9), HLA-DR-PE (LN3), H3-Pacific Blue, and H3K27me3-Alexa-488. The following color code applies: all events are colored black; all lymphocytes are colored red; T-cells (CD3+ CD19-) are colored dark blue; CD3- CD19- lymphocytes are colored yellow; NK cells are colored dark green; Dendritic cells are colored light blue; B-cells (CD19+CD3-) are colored purple; Granulocytes and monocytes are colored light green; monocytes (CD14+HLA-DR+) are colored orange; and granulocytes (CD14-HLA-DR-) are colored maroon. With respect to the right most panel, the tall left peak represents monocytes (dark blue) and the tall right peak represents T-cells (magenta).

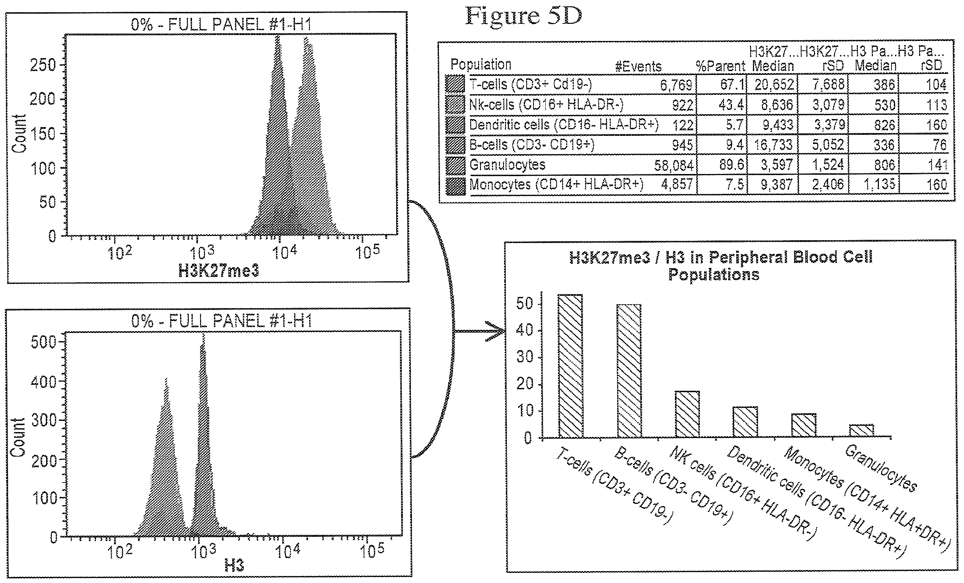

**Figure 5D** is a series of plots and a corresponding graph that depict the simultaneous detection by flow cytometry of cellular surface epitopes and nuclear epitopes in a blood cell sample using a panel of antibodies. The panel of antibodies represented on the flow cytometry plots is CD3-V500-C (SK7), CD19-BV421 (HIB19), CD16-PE-Cy7 (B73.1), CD14-PerCP-Cy5.5 (M_{Ψ}P9), HLA-DR-PE (LN3), H3-Alexa 647, and H3K27me3-Alexa-488. With respect to the top left panel, the tall left peak represents monocytes (dark blue) and the tall right peak represents T-cells (magenta). With respect to the top right panel, the tall right peak represents T-cells (magenta) and the tall right peak represents monocytes (dark blue).

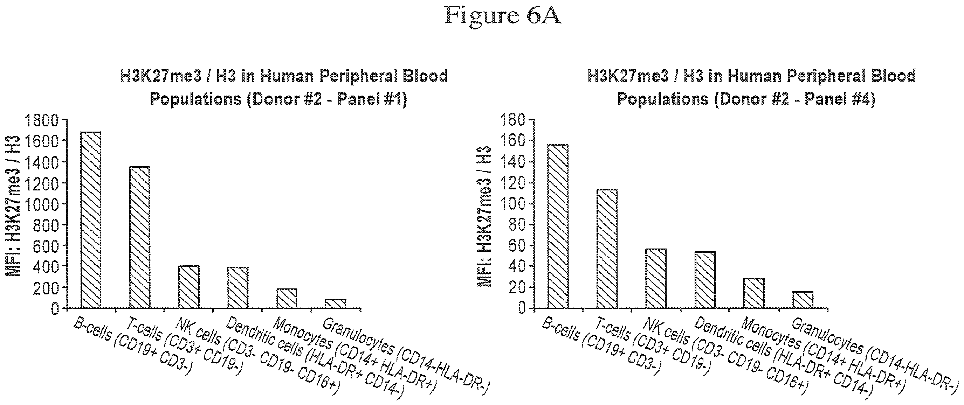

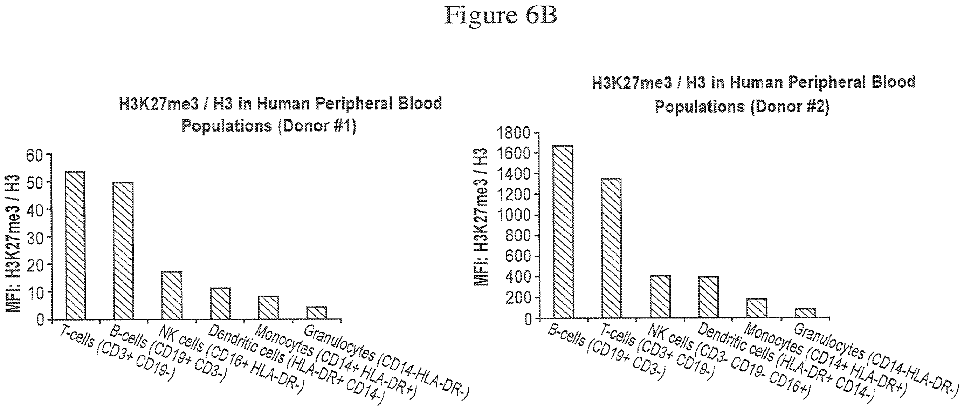

**Figure 6A** is a series of bar graphs that depict representative flow cytometry data obtained from human peripheral blood samples. Representative flow cytometry data were obtained from the same donor and processed with different antibody panels of antibodies. In the bar graph on the left side of Figure 6A the following panel of antibodies was used for the flow cytometry procedure: CD3-V500-C (SK7), CD19-APC (HIB19), CD16-PE-Cy7 (CD16), CD14-PerCP-Cy5.5 (M_{Ψ}P9), HLA-DR-PE (LN3), H3-Pacific Blue, and H3K27me3-Alexa-488. In the bar graph on the right side of Figure 6B the following panels of antibodies was used for the flow cytometry procedure: CD3-V500-C (SK7), CD19-BV421 (HIB19), CD16-PE-Cy7 (B73.1), CD14-PerCP-Cy5.5 (M_{Ψ}P9), HLA-DR-PE (LN3), H3-Alexa 647, and H3K27me3-Alexa-488.
**Figure 6B** is a series of bar graphs that depict representative flow cytometry data obtained from human peripheral blood samples. Representative flow cytometry data were obtained from different donors processed with the same antibody panel.

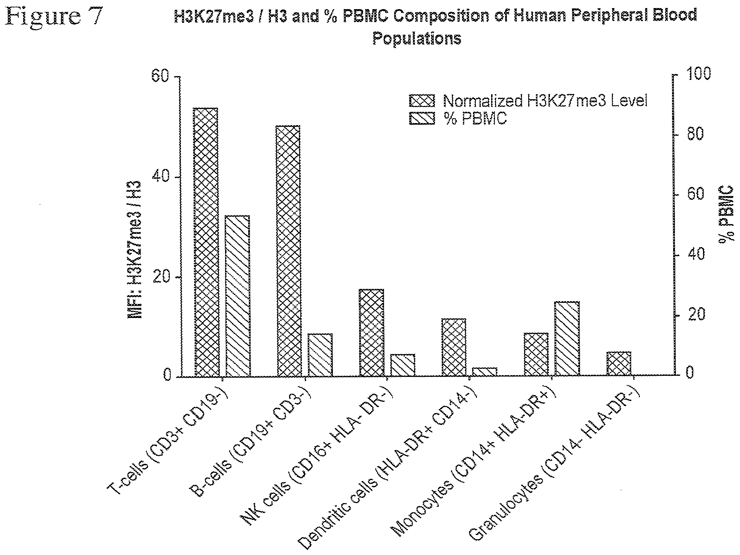

**Figure 7** is a graph that depicts flow cytometry data obtained from a blood sample contacted with cell surface specific antibodies, antibodies to H3K27me3 and histone H3. The graph in Figure 7 depicts a comparison between blood cell type and H3K27me3 status.

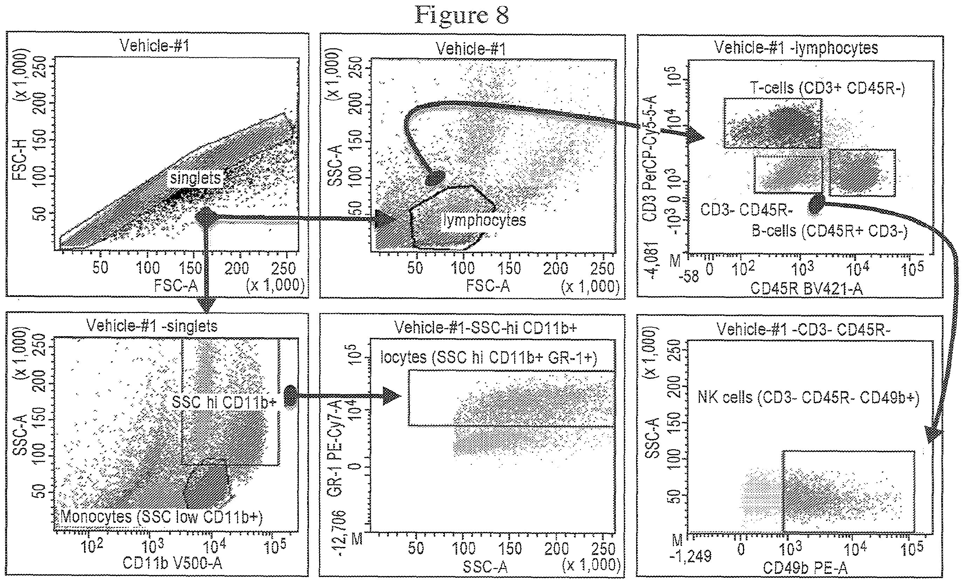

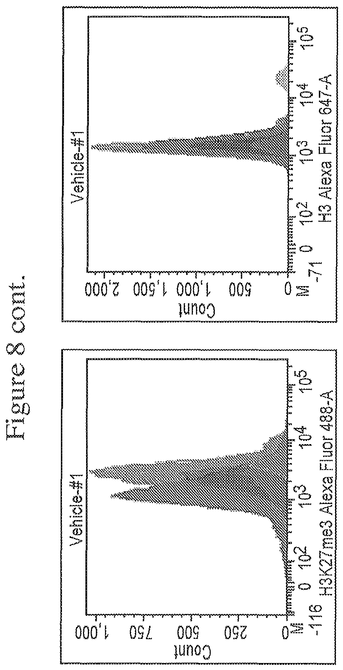

**Figure 8** is a series of flow cytometry plots that depict the results obtained from an *in vivo* rodent dose response study which purpose was to ascertain the effect of administration of the EZH2 inhibitor EPZ-6438. The flow cytometry data plots in Figure 8 depict data obtained from a blood sample acquired from a rodent which had received a control, vehicle-only treatment.

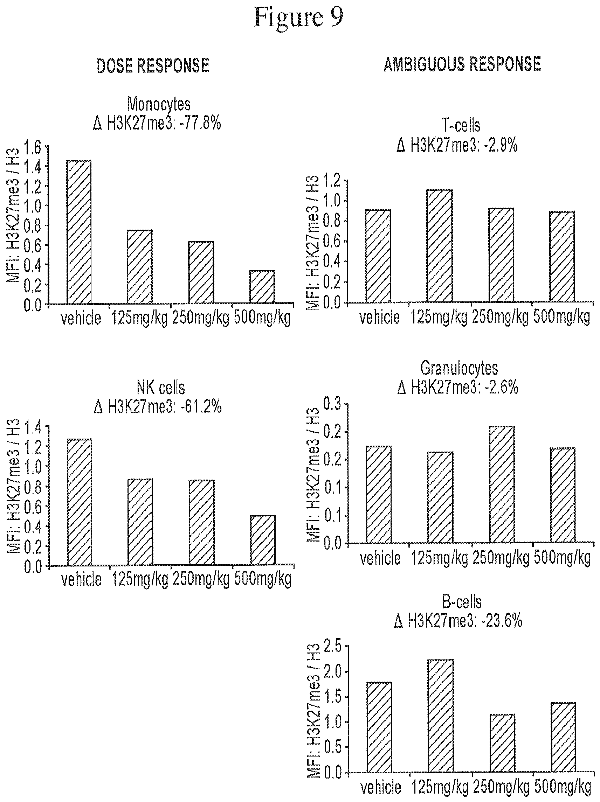

**Figure 9** is a series of bar graphs that depict mean fluorescent intensity (MFI) data obtained from flow cytometry analysis of samples from a rodent *in vivo* dose response study in which the rodents received specified dosages of the EZH2 inhibitor EPZ-6438. The rodents in the study received a vehicle treatment, 125 mg/kg, 250 mg/kg, or 500 mg/kg of the EPZ-6438 compound. The data from this study is presented as a series of bar graphs obtained from flow cytometry assays.

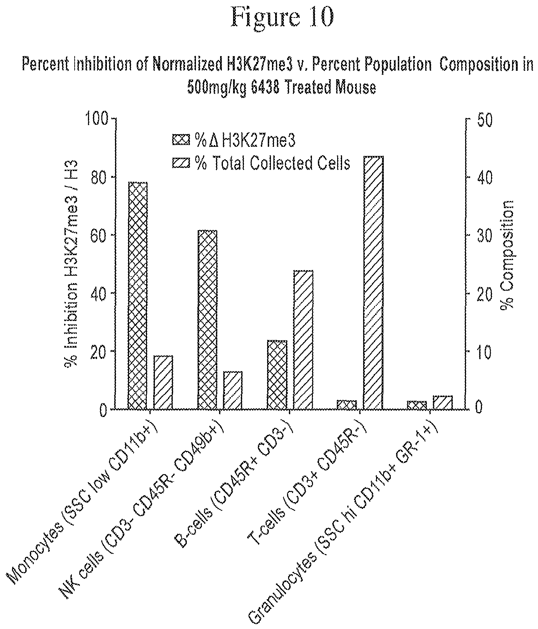

**Figure 10** is a bar graph obtained from flow cytometry data that depicts the contribution of discrete cellular populations to H3K27me3 signal following treatment of a mouse with 500 mg/kg of EPZ-6438. The surface markers used in the flow cytometry assay allowed for the determination of H3K27me3 status in monocytes, NK cells, B-cells, T cells and granulocytes.

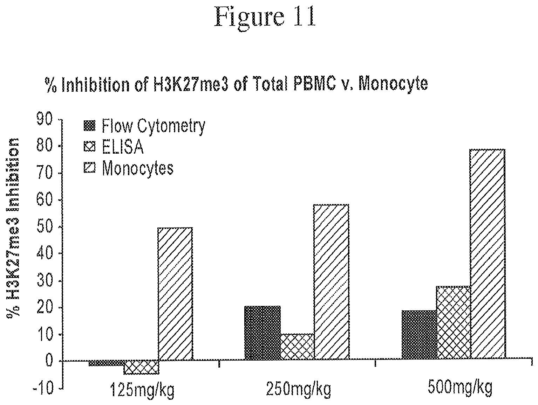

**Figure 11** is a bar graph that depicts the percentage H3K27me3 inhibition following administration of various concentrations of EPZ-6438 as assessed by either a flow cytometry assay or ELISA. The amounts of H3K27me3 inhibition in total PBMC is compared with H3K27me3 inhibition of monocytes. The concentrations of the EPZ-6438 administered to the rodents in the study was 125 mg/kg, 250 mg/kg, or 500 mg/kg. Flow cytometry represented by black bars, ELISA represented by red bars and monocytes represented by blue bars.

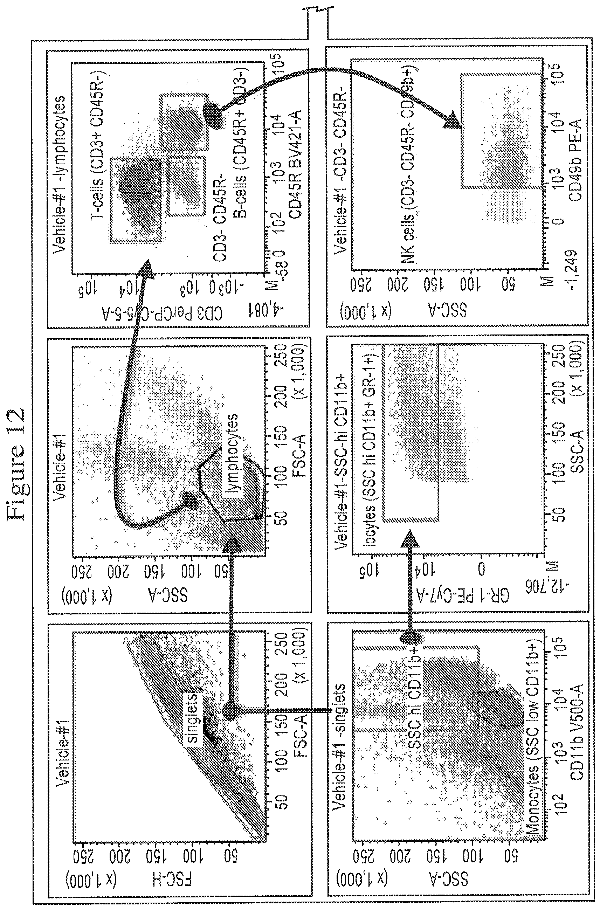

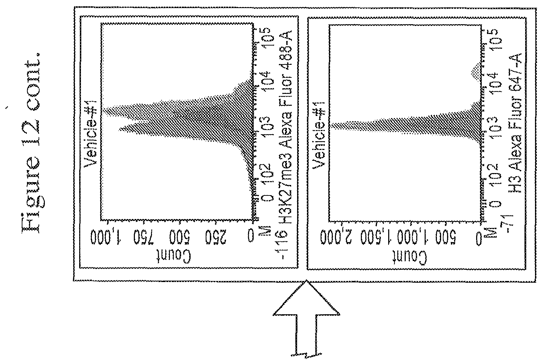

**Figure 12** is a collection of graphs depicting a gating scheme for mouse peripheral blood following FACS.

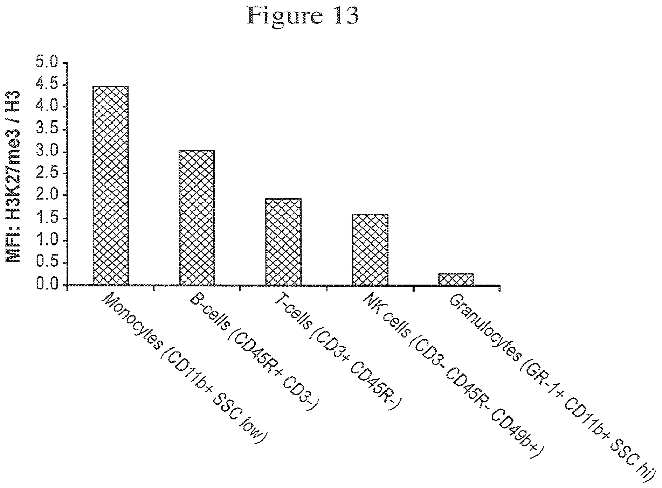

**Figure 13** is a graph depicting the prevalence of the H3K27me3 methyl mark in mouse peripheral blood populations.

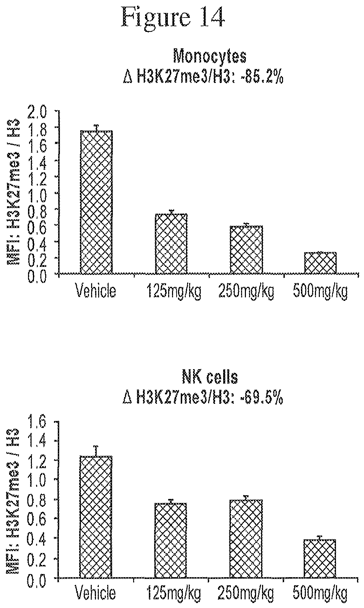

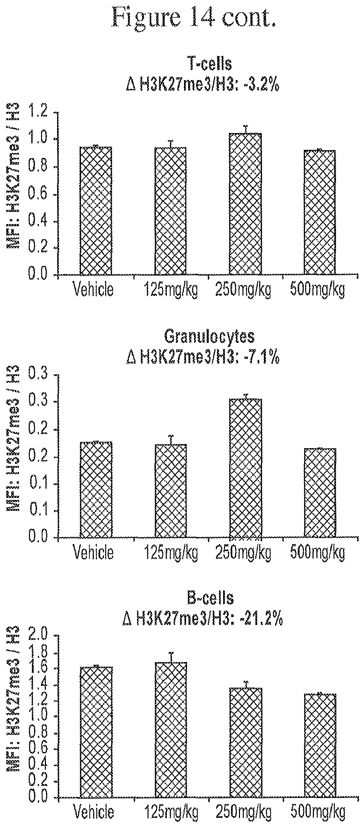

**Figure 14** is a series of graphs depicting differential sensitivity of mouse leukocyte populations to tazemetostat

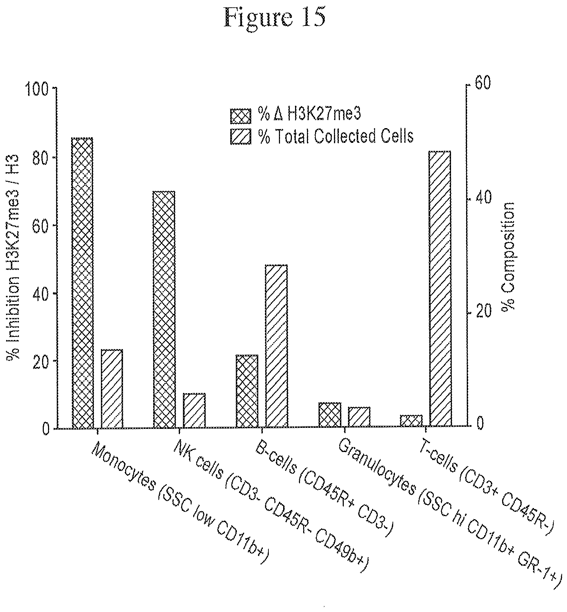

**Figure 15** is a graph depicting cell type contribution to total H3K27me3 inhibition.

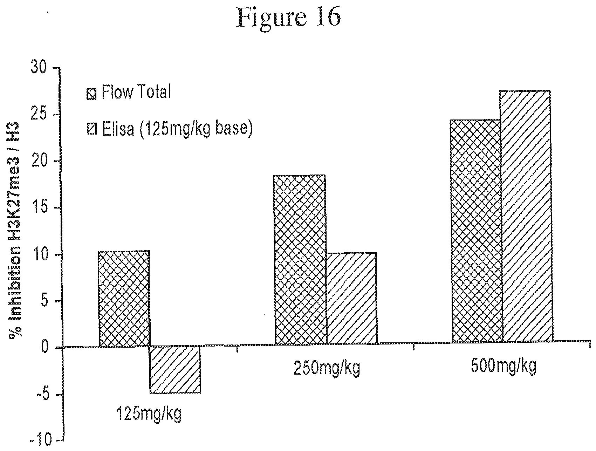

**Figure 16** is a graph depicting H3K27me3 quantification from chromatin flow cytometry versus ELISA.

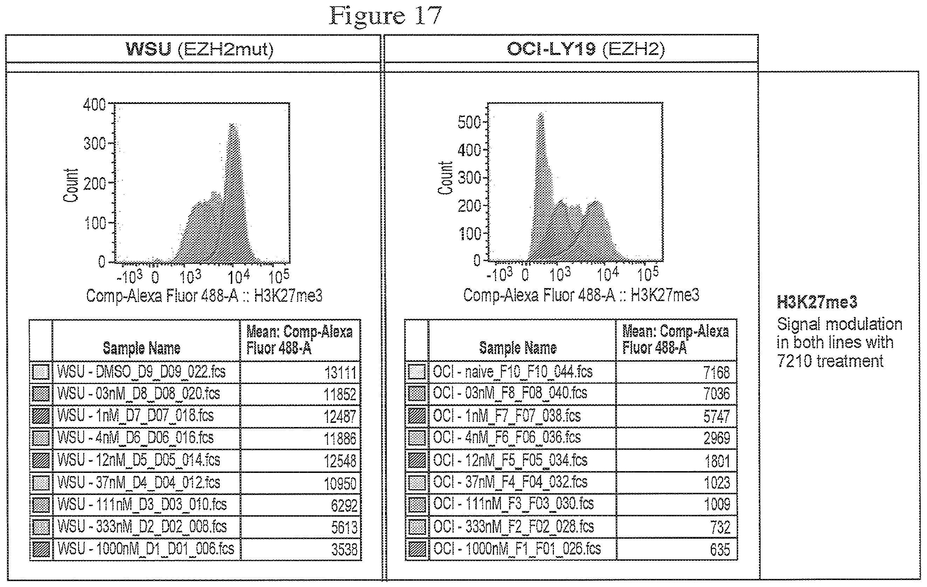

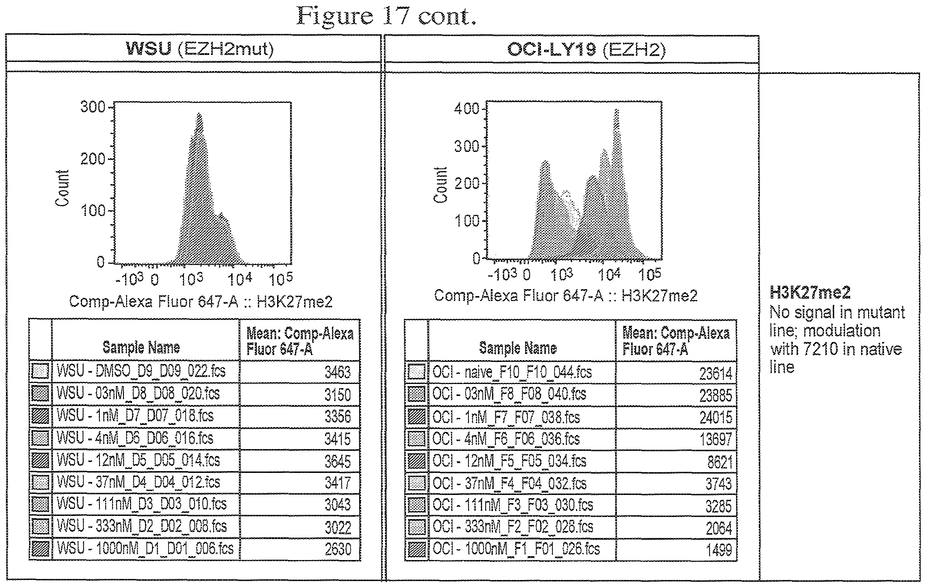

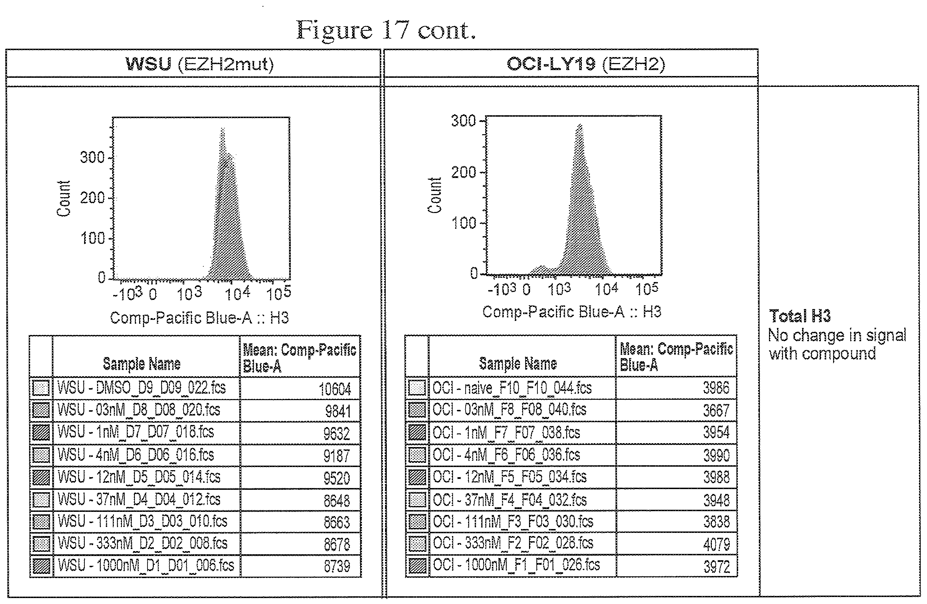

**Figure 17** is a series of plots depicting dose response to EPZ007210 in EZH2 mutant and wild type cell lines. H3K27me3 status is depicted on the flow cytometry plots following incubation with the following EPZ007210 concentrations DMSO only (yellow), 0.3nM (light blue), 1nM (magenta), 4nM (pink), 12nM (dark green), 37nM (light green), 111nM (orange), 333nM (teal) and 1000nM (red).

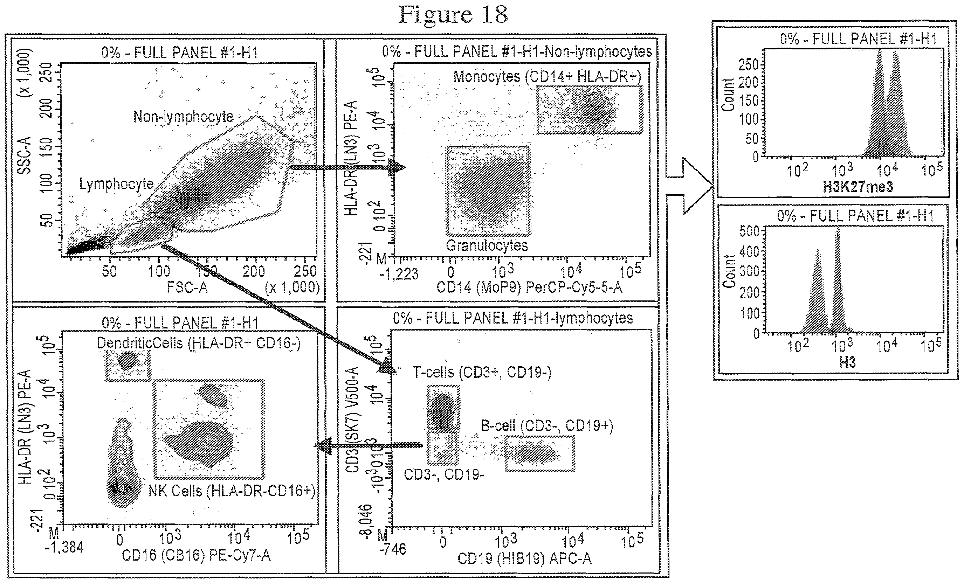

**Figure 18** is a series of plots depicting a gating scheme for human peripheral blood following FACS. The following color scheme applies: Granulocytes are colored red, monocytes are colored blue, T cells are colored blue, and B-cells are colored magenta.

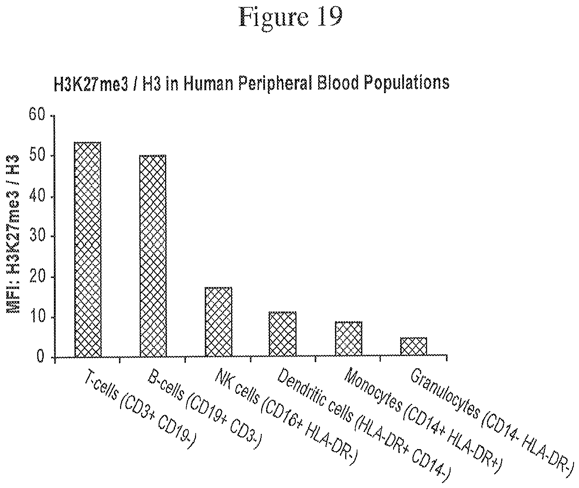

**Figure 19** is a graph depicting the relative prevalence of the H3K27me3 methyl mark in human peripheral blood populations.

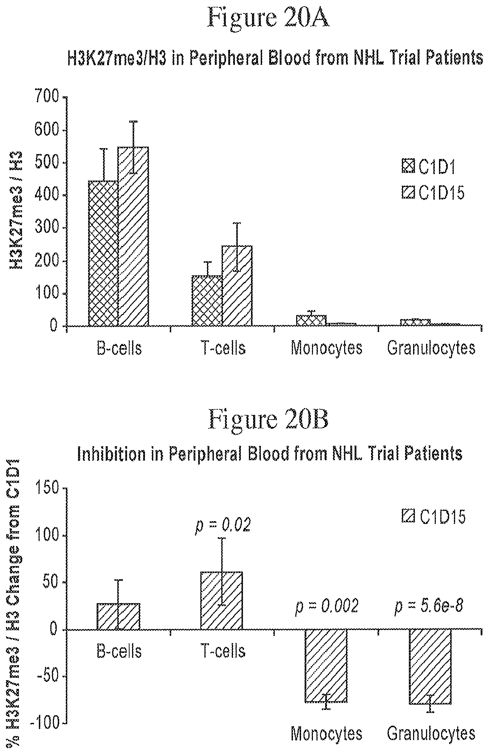

**Figure 20** is a pair of graphs depicting H3K27me3 inhibition in a NHL trial with tazemetostat treatment.

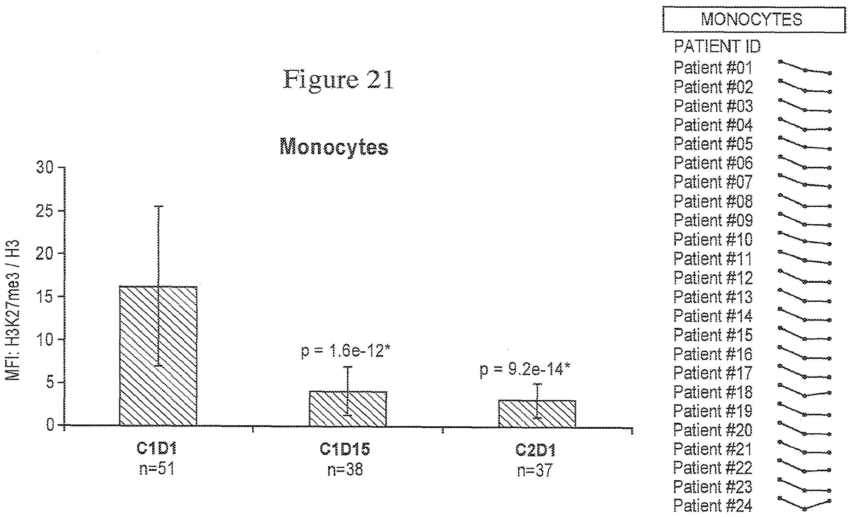

**Figure 21** is a graph depicting Phase II pharmacodynamics for monocytes isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1).

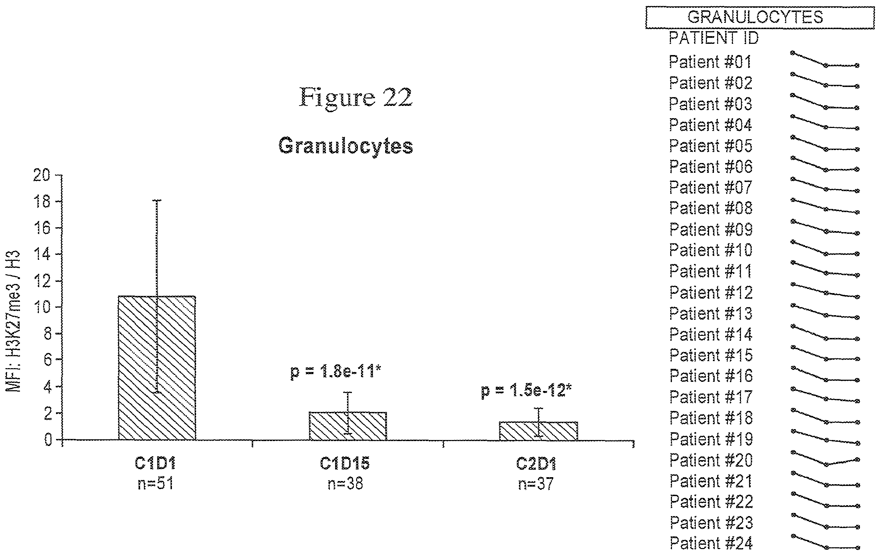

**Figure 22** is a graph depicting Phase II pharmacodynamics for granulocytes isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1).

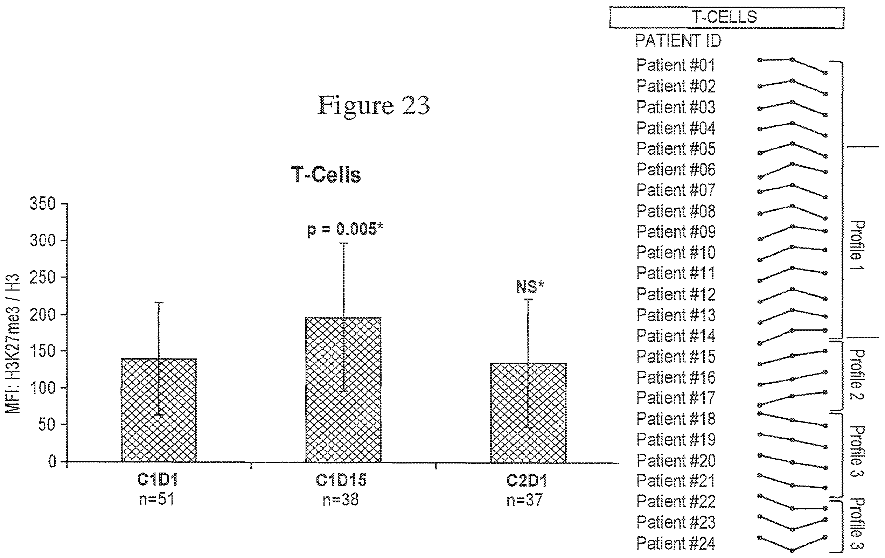

**Figure 23** is a graph depicting Phase II pharmacodynamics for T-cells isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1). T-cells demonstrate a modest, but statistically significant increase in H3K37me3 at day 15 (cycle 1 day 15) of tazemetostat exposure followed by a return to baseline at day 30 (cycle 2, day 1).

### DETAILED DESCRIPTION

### Introduction

Epigenetic modification of chromatin structures is a mechanism through which gene expression is regulated. Chromatin is composed of DNA, RNA and protein (*e.g*. histones) complexes. Epigenetic modifications include, among other processes, acetylation and methylation of chromatin. Links between aberrant epigenetic modification and various kinds of disease have previously been presented. Diseases that are associated with changes in epigenetic modifications include various kinds of cancer, neurodegenerative disorders, inflammatory conditions, and oral disease.

Modification of histones through various mechanisms (*e.g.* methylation, acetylation, phosphorylation and ubiquitinylation) is involved in epigenetic regulation. Histones are categorized into five families. These families of histones are H1/H5, H2A, H2B, H3 and H4. These histone families are further categorized as core histones (H2A, H2B, H3, and H4), and as linker histones (HI and H5). The core histones assemble into a set of eight proteins to create the histone octomer around which DNA wraps. The histone octomers are composed of two copies of each of the core histones. Modification of histones (*e.g.* through methylation via HMTs) is associated with changes in gene expression. Histone methyltransferases (HMTs) form a part of the regulatory system that controls gene expression. HMTs regulate gene expression through the placement of methyl marks (*e.g.,* one, two, or three methyl groups) onto specific histone amino acids, namely lysine and arginine residues. Genetic alterations may alter HMT activity and, as a consequence, make the HMTs oncogenic due to misregulated gene expression, and resultant aberrant placement of methyl marks onto histones.
Previous methods for detecting epigenetic marks, *e.g.,* histone methylation marks, did not allow for the meaningful detection and monitoring of such epigenetic marks during the treatment of a subject with an epigenetic modulator (*e.g*., tazemetostat). Some aspects of the present disclosure relate to the recognition that different cell types react differently to administration of an epigenetic modulator. Unlike previous methods, the methods of the present disclosure account for this variation, allowing for accurate and meaningful determination and monitoring of epigenetic marks in isolated, enriched, or purified cell populations. In some embodiments, the methods of the disclosure provide cost and time efficient analyses that can be used in a clinical setting, *e.g.,* by isolating subpopulations of cells from a subject or a sample obtained from a subject and measuring epigenetic markers in such subpopulations. In some embodiments, the methods of the disclosure incorporate cell sorting procedures, *e.g.,* by fluorescent-activated cell sorting or magnetic cell sorting techniques, to isolate subpopulations of cells and measuring epigenetic markers in such subpopulations. In some embodiments, the methods of the disclosure are useful for assessing epigenetic marks in a subject to diagnose a disease or an epigenetic state associated with a disease, to determine whether a subject is a candidate for a treatment with an epigenetic modulator, and/or to monitor the effect of an epigenetic modulator, such as, *e.g.,* a histone methyltransferase inhibitor, administered to a subject on the epigenetic state on a target population of cells, *e.g.,* on histone methylation in a subpopulation of blood cells, in the subject.

### Methods for determining epigenetic biomarkers

While the disclosure provides working examples for the detection of H3 methylation marks, the methods provided herein are not limited to this biomarker. The methods, strategies, and assays of the disclosure may be used to monitor a status of any epigenetic modification (e.g. methylation, acetylation, phosphorylation, and ubiquitinylation) that can be detected.

In some embodiments, the methods provided herein provide a means of monitoring a status of any epigenetic modification in a target cell that is known or suspected to react to administration of an epigenetic modulator. In some such embodiments, the methods, strategies, and assays of the disclosure include a step of isolating a cell population of interest and measuring an epigenetic mark, *e.g.,* a histone methylation mark, in that enriched, isolated, or purified cell population.

In some embodiments, a target population of cells may be identified by one or more physical characteristics or biomarkers, including, but not limited to, cell shape, size, cell surface protein expression (including, but not limited to cell surface antigens), epigenetic modification, or any combination thereof. Epigenetic and protein modifications may be used as biomarkers and may include, but may not be limited to, methylation, acetylation, ubiquitination, *e.g.,* of genomic or protein sequences. Epigenetic and protein modifications may be measured in absolute (*e.g.,* present/absent) or in relative terms (*e.g.,* ratio of modified to unmodified sites or proteins; ratio of modified sites or protein to total sites/total available sites or total protein; ratio of modified sites or protein per cell or number of modified cells per subpopulation or per cell; ratio of modified sites or protein compared to an external standard (*e.g.,* a known threshold for indicating or predicting cancer risk); or any combination thereof. It will be understood that the biomarkers listed above are meant to be exemplary and do not limit the present disclosure. Additional suitable biomarkers will be apparent to those of ordinary skill in the art based on the present disclosure and the knowledge in the art.

A target cell population may comprise any suitable cell type, including, but not limited to, cells isolated from blood, *e.g.,* from peripheral blood, or from a population of peripheral blood mononuclear cells, lymphoid cells, myeloid cells, myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells; or any combination thereof. In some embodiments, a target cell is characterized by the presence or absence of a cell surface antigens, or of a combination of cell surface antigens. Exemplary cell surface antigens include, but are not limited to, CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof.

In some embodiments, a target cell population comprises a subpopulation of cells isolated from a biological sample or a cell population comprising the subpopulation in addition to other cells. In some embodiments, a subpopulation of cells may be isolated or identified by cell sorting techniques, *e.g.,* by magnetic cell sorting or by flow cytometry. Exemplary suitable cell sorting techniques include, but are not limited to, fluorescent-activated cell sorting (FACS) and magnetic-activated cell sorting (MACS).

Methods, strategies, and assays of the disclosure may comprise one or more steps for isolating, sorting, and/or purifying cell populations or subpopulations until these populations contain at least 50%, at least 60%, at least 70%, at least 80%, at least 90%, at least 95%, at least 99%, or 100% cells of interest, also referred to herein as target cells, or any percentage in between. Cells of interest may present one or more physical characteristics or biomarkers of interest. Alternatively, or in addition, cells of interest may be sensitive to treatment with an epigenetic modulator or may be isolated or derived from a tissue or a subject that is sensitive to treatment with an epigenetic modulator. In some embodiments, a population of target cells that is sensitive to treatment with an epigenetic modulator is isolated from a larger population of cells that comprises the target cell population and other cells that are not sensitive to such treatment or cells that show lesser sensitivity than the target cell population or a mixed response to such treatment.

Methods, strategies, and assays of the disclosure may be used to identify cells, tissues, and/or subjects that are sensitive to treatment with an epigenetic modulator.
Methods, strategies, and assays of the disclosure may be used to monitor the state of an epigenetic mark, *e.g.,* the status of histone methylation, in a subject during treatment with an epigenetic modulator, *e.g.,* a subject receiving an EZH2 inhibitor (*e.g.,* tazemetostat). Methods, strategies, and assays of the disclosure may comprise the steps of determining a first level of an epigenetic mark in a cell or tissue obtained from a subject before treatment of the subject with an epigenetic modulator (including tazemetostat), administrating a therapeutically effective amount of the epigenetic modulator and determining a second level of an epigenetic mark in a cell or tissue obtained from a subject following treatment with the epigenetic modulator. This method may be repeated one or more times following each treatment. For example, a second and/or subsequent level of an epigenetic mark in a cell or tissue obtained from a subject may be determined every day, every other day, every week, every other week, every month, or every other month following a treatment with an epigenetic modulator. In certain embodiments, a second and/or subsequent level of an epigenetic mark in a cell or tissue obtained from a subject may be determined every 2, 4, 6, 8, 10, 12, 14, 16, 18, 20, 22, or 24 hours; every 1, 2, 3, 4, 5, 6, 7, 8, 9, 10, 11, 12, 13, or 14 days; every 1, 2, 3, 4, 5, 6, 7, 8,9, 10, or 12, weeks; every 1, 2, 3, 4, 5, or 6 months, or every year following a treatment with an epigenetic modulator.

Methods, strategies, and assays of the disclosure may be used to monitor a subject during treatment with an epigenetic modulator, *e.g.,* a subject receiving an EZH2 inhibitor (*e.g.,* tazemetostat) until a particular treatment outcome has been achieved. For example, methods, strategies, and assays of the disclosure may be used to monitor a subject during treatment with an epigenetic modulator, *e.g.,* a subject receiving an EZH2 inhibitor (*e.g.,* tazemetostat) until a second or subsequent level of an epigenetic modification has been downregulated or decreased by at least 50%, at least 60%, at least 70%, at least 80%, at least 90%, at least 95%, at least 99%, 100% compared to a first level of an epigenetic modification. Methods, strategies, and assays of the disclosure may be used to monitor a subject during treatment with an epigenetic modulator, *e.g.,* a subject receiving an EZH2 inhibitor (*e.g.,* tazemetostat) until the subject has entered a period of remission from a disease (*e.g.,* cancer). Methods, strategies, and assays of the disclosure may be used to monitor a subject during treatment with an epigenetic modulator, *e.g.,* a subject receiving an EZH2 inhibitor (*e.g.,* tazemetostat) until the subject has been symptom-free of a disease or disorder for a period of at least 1, 2, 3, 4, 5, 6, 7, 8, ,9 10, 11 or 12, weeks; 1, 2, 3, 4, 5, or 6 months; or 1 year.

In certain embodiments, a certain level of an epigenetic mark may be considered healthy or normal, whereas a deviation above or below this "normal" level is considered pathologic. For example, in some embodiments, a suitable reference level characteristic for a "normal" epigenetic state is a level of an epigenetic mark measured in a healthy subject, an average level of an epigenetic mark measured across a population of subjects, e.g., a population of healthy subjects or a general population of subjects, or a historical or empirical value. Additional suitable reference levels will be apparent to those of ordinary skill in the art based on the instant disclosure and the disclosure is not limited in this respect. Methods, strategies, and assays of the disclosure may be used to monitor a subject during treatment with an epigenetic modulator, *e.g.,* a subject receiving an EZH2 inhibitor (*e.g.,* tazemetostat) until the level of the epigenetic modification in that subject has returned to a "normal" level from a pathologically high or low level. In certain embodiments, the "normal" level may be established my detecting a level of the epigenetic modification in an individual who is free of any sign or symptom of the condition of interest and who, optionally, has no medical history and/or genetic risk factor for developing the condition of interest.

### Exemplary suitable cell populations

Methods, strategies, and assays of the disclosure comprise cell populations that are isolated, enriched or purified to comprise predominantly one cell type or a collection of highly-related subtypes (*e.g.,* derived from a common stem cell progenitor or tissue type) that react more similarly to administration of an epigenetic modulator than a mixed population of cells (*e.g.,* a population of cells from a biopsy or biological sample prior to enrichment for cells having similar physical characteristics or displaying similar biomarkers). For example, cell populations of the methods, strategies, and assays of the disclosure may be isolated, enriched or purified to comprise, consist essentially, or consist of lymphoid cells. In some embodiments, cell populations of the methods, strategies, and assays of the disclosure may be isolated, enriched or purified to comprise, consist essentially, or consist of myeloid cells. In some embodiments, cell populations of the methods, strategies, and assays of the disclosure may be isolated, enriched or purified to comprise, consist essentially, or consist of myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells, or any combination thereof. In some embodiments, the population of cells may be isolated, enriched or purified from peripheral blood or from a population of peripheral blood mononuclear cells obtained from the subject.

Cell populations of the methods, strategies, and assays of the disclosure may be isolated, enriched or purified to comprise, consist essentially of, or consist of cells characterized by expression of a cell surface antigen or a combination of cell surface antigens. In some embodiments, the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof. In some embodiments, the cell surface antigen is CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR, or any combination thereof.

Cell populations of the methods, strategies, and assays of the disclosure may be isolated, enriched or purified to comprise, consist essentially, or consist of myeloid stem or progenitor cells that are CD133⁺, CD271⁺, CD11⁺, and/or CD347⁺; erythroblasts that are CD71⁺; megakaryocytes that are CD61⁺; platelets that are CD61⁺; granulocytes that are HLA-DR⁺ and CD14⁻; basophils that are CD123⁺; eosinophils that are CD44⁺; neutrophils that are CD15⁺ and/or CD16⁺; monocytes that are CD14⁺, CD11b⁺, CD16⁻, and/or HLA-DR⁺; MDClc cells that are CD1c⁺; MDC2 cells that are CD141⁺; mast cells that are CD117⁺; lymphoid stem or progenitor cells that are CD34⁺, CD133⁺, CD271⁺, CD117⁺; T-lymphocytes (T-cells) that are CD2⁺ and/or CD3⁺; cytotoxic T cells that are CD8⁺; T helper cell that CD4⁺; regulatory T-cells that are CD4⁺ and CD25⁺; NK/T cell that are CD3⁺ and CD56⁺; natural killer T cells that are CD56⁺; activated T cells that are CD25⁺ and CD30⁺; B-cells that are CD19⁺ and/or CD20⁺; activated B-cells that are CD19⁺, CD25⁺, and/or CD30⁺; plasma cells that are CD138⁺; natural killer B cells that are CD56⁺; natural killer (NK) cells that are CD3⁻, CD19⁻, HLA-DR⁺ and CD16⁻; plasmacytoid dendritic cells that are CD304⁺; or any combination thereof.

Cell populations of the methods, strategies, and assays of the disclosure may be isolated, enriched or purified to comprise, consist essentially, or consist of B-cells.

Cell populations of the methods, strategies, and assays of the disclosure may be isolated, enriched or purified to comprise, consist essentially, or consist of T cells.

Cell populations of the methods, strategies, and assays of the disclosure may be isolated, enriched or purified to comprise, consist essentially, or consist of monocytes. In some embodiments, the monocytes are CD14⁺ and CD16⁻; CD14^{low} and CD16⁺; or CD14^{high} and CD16^{low} monocytes, or any combination thereof.

Cell populations of the methods, strategies, and assays of the disclosure may be isolated, enriched or purified to comprise, consist essentially, or consist of granulocytes. In some embodiments, the granulocytes are neutrophils, eosinophils, or mast cells, or any combination thereof.

Cell populations of the methods, strategies, and assays of the disclosure may be isolated, enriched or purified to comprise, consist essentially, or consist of at least 50%, at least 60%, at least 70%, at least 80%, at least 90%, at least 95%, at least 99%, 100% cells of interest or any percentage in between. Cells of interest may present one or more physical characteristics or biomarkers of interest.

Additional suitable cell populations will be apparent to those of ordinary skill in the art based on the instant disclosure, and suitable cell surface markers for isolating suitable cell populations of suitable purity will be known to those of ordinary skill in the art. Target cell populations can be identified, isolated, and/or purified by any suitable cell sorting technique known in the art. Suitable cell sorting techniques and cell surface markers characteristic for some exemplary suitable target cell populations may include, in some embodiments, those described in one or more of the following, the contents of each of which are incorporated by reference in their entirety: Flow Cytometry Protocols (Methods in Molecular Biology) 3rd ed. 2011; by Teresa S. Hawley (Editor), and Robert G. Hawley (Editor); ISBN-10: 1617379492; Flow Cytometry: Principles and Applications, 2007, by Marion G. Macey (Editor); ISBN-10: 1588296911; T Cell Protocols (Methods in Molecular Biology), 2008, by Gennaro De Libero (Editor); ISBN-10: 1588295877; Immunophenotyping, 1st Edition, by Carleton C. Stewart (Editor), Janet K. A. Nicholson (Editor); ISBN-10: 0471239577; Flow Cytometry and Cell Sorting (Springer Lab Manuals) 2nd Edition, 2000; by Andreas Radbruch (Editor); ISBN-10: 3642084923; A List of exemplary suitable CD antigens and CD antigen profiles for various cell types is provided in Appendix II, CD Antigens, of Immunobiology: The Immune System in Health and Disease, 5th edition., by Janeway CA Jr, Travers P, Walport M, et al.; New York: Garland Science; 2001.; ISBN-10: 081533642X.

### EZH2

EZH2 is an exemplary histone methyltransferase of the disclosure. Although the disclosure provides a detailed analysis of the mechanisms and uses for certain epigenetic modulators (*e.g.,* EZH2 inhibitors and, in particular tazemetostat), the disclosure is not limited to methylation marks, EZH2 inhibitors, or tazemetostat. The methods, strategies, and assays described herein may be applied to any epigenetic modification and may be used to monitor the progress of any epigenetic modulator.

EZH2, a histone methyltransferase, has been associated with various kinds of cancers. Specifically, mutations and and/or overactivity of EZH2 are found in a range of cancers, such as lymphomas, leukemias and breast cancer. There is an ongoing need for new agents as EZH2 inhibitors for use in anticancer treatment, as well as for new methods to assess epigenetic modification in a mixed cell population.

EZH2 is a histone methyltransferase that is the catalytic subunit of the PRC2 complex which catalyzes the mono- through tri-methylation of lysine 27 on histone H3 (H3-K27). Histone H3-K27 trimethylation is a mechanism for suppressing transcription of specific genes that are proximal to the site of histone modification. This trimethylation is known to be a cancer marker with altered expression in cancer, such as prostate cancer (see, *e.g.,* U.S. Patent Application Publication No. 2003/0175736; incorporated herein by reference in its entirety). Other studies provided evidence for a functional link between dysregulated EZH2 expression, transcriptional repression, and neoplastic transformation. Varambally et al. (2002) Nature 419(6907):624-9 Kleer et al. (2003) Proc Natl Acad Sci USA 100(20):11606-11.

EZH2 methylation activity plays an important role in the regulation and activation of germinal center B-cells. EZH2 protein levels increase following the activation of B-cells. Following activation, B-cells take residence in the germinal center of lymphoid organs, wherein somatic hypermutation occurs, a process associated with the repression of anti-apoptotic genes and check point regulators. EZH2 methylating events target genes that are involved in B-cell proliferation, differentiation and maturation, including CDKN1A (role in cellular proliferation), PRDM1 (role in B-cell differentiation) and IRF4 (role in B-cell differentiation).

Following the maturation and exit of B-cells from the germinal center, there is a reduction of the levels of EZH2 within the B-cells. However, EZH2 presence and activity after B-cell maturation is associated with several kinds of lymphomas including germinal center B-cell lymphoma, among others.

Aberrant activation or misregulation of EZH2 is found in many common subtypes of non-Hodgkin lymphoma (NHL): diffuse large B cell lymphoma (DLBCL), germinal center B-cell like diffuse large B-cell lymphoma (GCB DLBCL), non-germinal center B-cell like diffuse large B-cell lymphoma including activated-B cell lymphoma (ABC DLBCL), Burkitt's lymphoma and other subtypes of non-Hodgkin lymphoma. Aberrant activation of or misregulation EZH2 is also found in follicular lymphoma (FL), Primary Mediastinal Large B-Cell Lymphoma (PMBCL) and marginal zone lymphoma (MZL).

Several kinds of EZH2 activating mutations have been described. EZH2 activating mutations result in the trimethylation of histone 3 lysine 27 (H3K27me3) resulting in the silencing of several tumor suppressor genes. In certain embodiments, a Y641 mutant of human EZH2, and, equivalently, a Y641 mutant of EZH2, refers to one kind of activating EZH2 mutation in which the amino acid residue corresponding to Y641 of wild-type human EZH2 is substituted by an amino acid residue other than tyrosine.

Although subjects having wild type EZH2 genes may have the H3K27me3 methylation mark, genetic alterations within the EZH2 gene are associated with altered histone methylation patterns. EZH2 mutations leading to the conversion of amino acid Y641 (equivalent to Y646, catalytic domain), to F, N, H, S or C results in hypertrimethylation of H3K27 and drives lymphomagenesis. Additional genetic alterations that affect the methylation of H3K27 include EZH2 SET-domain mutations, overexpression of EZH2, overexpression of other PRC2 subunits, loss of function mutations of histone acetyl transferases (HATs), and loss of function of MLL2. Cells that are heterozygous for EZH2 Y646 mutations result in hypertrimethylation of H3K27 relative to cells that are homozygous wild-type (WT) for the EZH2 protein, or to cells that are homozygous for the Y646 mutation.

Epigenetically modified histones of the methods of the disclosure may include, but are not limited to, H3K4me2/3, H3K79me3, H3K9me2/3, H3K27me2/3, H3K36me2/3 and/or H4K20me3. In certain embodiments, the epigenetically modified histone comprises H3K27me3.

Epitopes within an epigenetically modified histone of the methods of the disclosure may comprise at least one methylated amino acid residue. The methylated amino acid residue may comprise at least one methyl group. In certain embodiments, the methylated amino acid residue may comprise one methyl group. In certain embodiments, the methylated amino acid residue may comprise two methyl groups. In certain embodiments, the methylated amino acid residue may comprise three methyl groups.

Epitopes within the epigenetically modified histone of the methods described herein may be a non-linear epitope. Alternatively, or in addition, the epitope may be a conformational epitope. Alternatively, or in addition, the epitope may be a non-linear epitope. Alternatively, or in addition, the epitope may be a discontinuous epitope. Preferably, epitopes of the disclosure are human epitopes.

Epitopes within the epigenetically modified histone of the methods described herein may comprise at least one methylated lysine (K). The at least one methylated lysine (K) may be located in H3 or H4. In certain embodiments, the at least one methylated lysine (K) is located in H3. In certain embodiments, the at least one methylated lysine (K) is located in H4. For example, the one methylated lysine (K) may be H3K4me2/3, H3K79me3, H3K9me2/3, and/or H3K27me2/3. For example, the one methylated lysine (K) may be H4K20me3.

Chromatin flow cytometry is an effective means to monitor cell type specific changes in methylation state upon compound treatment *in vivo.* Chromatin flow cytometry has been validated in cell lines as a means to monitor epigenetic modifications with compound treatment. Mouse in vivo dose response studies and PD monitoring in human clinical trials establish chromatin flow cytometry as an effective means to quantify epigenetic modulation on discrete cell types. Given the advantages of this methodology over conventional measures of histone methylation, chromatin flow cytometry will be used to monitor pharmacodynamics of H3K27me3 inhibition in whole blood leukocytes in the ongoing phase 2 and future clinical trials of tazemetostat.

According to the methods of the disclosure, an activating EZH2 mutation may result in the trimethylation of H3K37 (also referred to as H3K27me3).

According to the methods of the disclosure, detecting levels of histone methylation includes, in some embodiments, detecting levels of H3K27 trimethylation (H3K27me3) in a biological sample from a subject. Histone methylation status may be detected prior to initiation of a treatment, while the subject is receiving treatment, and/or after treatment has concluded.

According to the methods of the disclosure, in certain embodiments of the disclosure the level of the H3K27me3 is compared and/or normalized by comparison with the level of the total H in the sample. In certain embodiments of the disclosure the step of comparison/normalization enhances the stability and/or clarity of the signal. In certain embodiments of the disclosure the step of comparison/normalization is essential for enhancement of the stability of the signal. In certain embodiments of the disclosure the step of comparison/normalization allows for accurate comparisons between intra and inter-assay methylation measurements.

According to the methods of the disclosure, a mutant EZH2 may comprise, consist essentially of or consist of a polypeptide sequence or a nucleic acid sequence encoding a mutant EZH2 polypeptide. In certain embodiments, the mutant EZH2 comprises one or more mutations in its substrate pocket domain. For example, the mutation may be a substitution, a point mutation, a nonsense mutation, a missense mutation, a deletion, or an insertion. Methods for detecting EZH2 mutations are further described in WO 2012/034132, WO 2013/138361, US 2015-0099747, the contents of each of which are incorporated herein by reference in its entirety.

Methods of the disclosure may comprise the step of administering a therapeutically effective amount of an EZH2 inhibitor to the subject. Alternatively, or in addition, the disclosure provides methods for monitoring the epigenetic status of H3K27 following administration of the EZH2 inhibitor. Thus, methods of the disclosure may comprise detecting the H2K27me3 status of a subject having cancer prior to and/or following treatment with an EZH2 inhibitor and comparing the result of the detection prior to treatment with the result of the detection following treatment, wherein a decrease of detection indicates an improved status of the subject and verifies the dose of the EZH2 inhibitor administrated to the subject as therapeutically-effective.

The EZH2 inhibitor suitable for the methods of the disclosure may be: 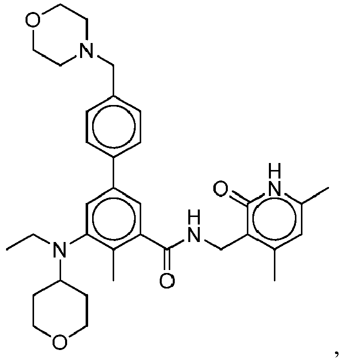 (also referred to as EPZ-6438 or tazemetostat) or a pharmaceutically acceptable salt thereof.

Preferably, subjects of the methods of the disclosure are human; however, the subject may be any species. Subjects may be either male or female. Subjects may be any age. In certain embodiments, a human subject is 40 years old or younger, 30 years old or younger, 20 years old or younger, or 10 years old or younger. In certain embodiments, a human subject is 17 years old or younger.

Subjects of the methods of the disclosure may have cancer. In certain embodiments, the cancer may be lymphoma. Exemplary forms of lymphoma include, but are not limited to, diffuse large B-cell lymphoma (DLBCL), a germinal center-derived lymphoma, a non-germinal center-derived lymphoma, follicular lymphoma (FL), primary mediastinal large B-cell lymphoma (PMBCL), marginal zone lymphoma (MZL), Burkitt's lymphoma and other non-Hodgkin's lymphoma subtype.

### EPZ-6438 (Tazemetostat)

EZH2 inhibitors of the methods comprise, consist essentially of or consist of EPZ-6438 (tazemetostat): 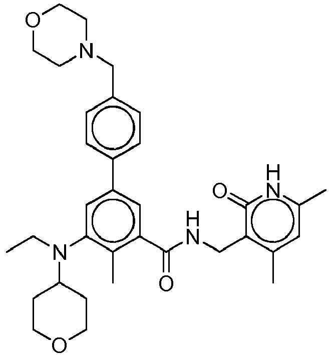 or a pharmaceutically acceptable salt thereof. Compositions for administration to a subject of the methods of the disclosure may comprise, consist essentially of or consist of EPZ-6438 (tazemetostat) or a pharmaceutically acceptable salt thereof, and a pharmaceutically-acceptable carrier.

EPZ-6438 or a pharmaceutically acceptable salt thereof, as described herein, is potent in targeting both WT and mutant EZH2. EPZ-6438 is orally bioavailable and has high selectivity to EZH2 compared with other histone methyltransferases (>20,000 fold selectivity by Ki). Importantly, EPZ-6438 has target methyl mark inhibition that results in the killing of genetically defined cancer cells *in vitro.* Animal models have also shown sustained *in vivo* efficacy following inhibition of target methyl mark. Clinical trial results described herein also demonstrate the safety and efficacy of EPZ-6438.

EPZ-6438 or a pharmaceutically acceptable salt thereof may be administered to the subject at a dose of approximately 100 mg to approximately 3200 mg daily, such as about 100 mg BID to about 1600mg BID (*e.g.,* 100 mg BID, 200 mg BID, 400 mg BID, 800 mg BID, or 1600 mg BID). EPZ-6438 or a pharmaceutically acceptable salt thereof may be administered to the subject at a dose of approximately 100 mg to approximately 3200 mg daily, such as about 100 mg BID to about 1600mg BID (*e.g.,* 100 mg BID, 200 mg BID, 400 mg BID, 800 mg BID, or 1600 mg BID) for treating a NHL. In certain embodiments of the methods of the disclosure, the dose is 800 mg BID.

In some embodiments, an EZH2 inhibitor that can be used in any methods presented here is any of Compounds A through D: 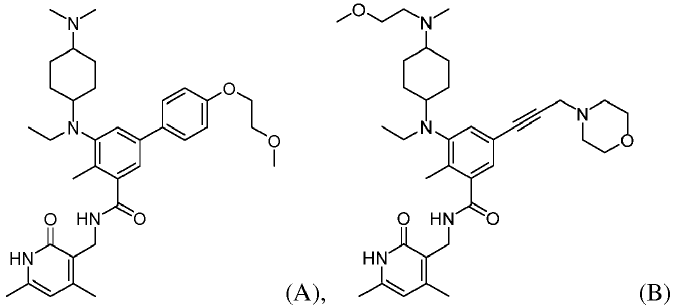 or 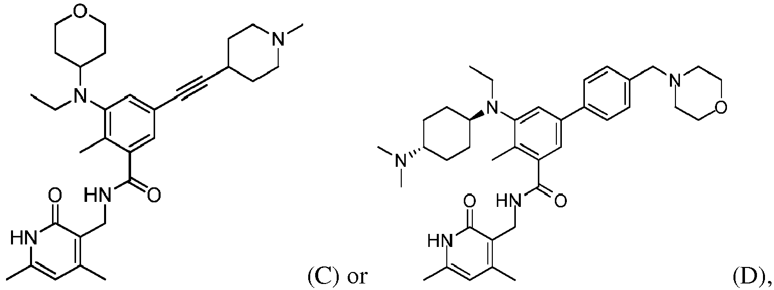 or stereoisomers thereof or pharmaceutically acceptable salts and solvates thereof. Compound D is also referred to as EPZ007210 herein.

In certain embodiments, an EZH2 inhibitor that can be used in any methods presented here is Compound E: 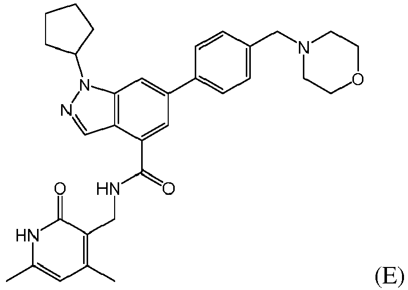 or pharmaceutically acceptable salts thereof.

In some embodiments, an EZH2 inhibitor that can be used in any methods presented here is GSK-126 having the following formula: 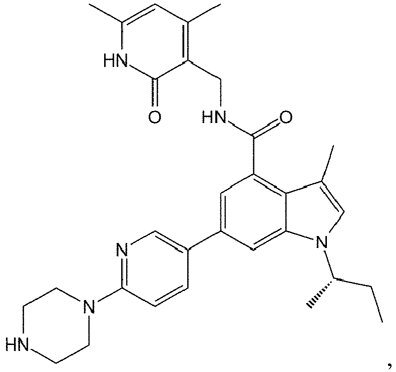 stereoisomers thereof, or pharmaceutically acceptable salts or solvates thereof.

In certain embodiments, an EZH2 inhibitor that can be used in any methods presented here is Compound F: 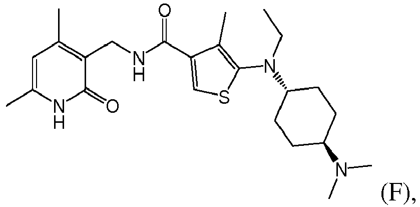 or stereoisomers thereof or pharmaceutically acceptable salts and solvates thereof.

In certain embodiments, an EZH2 inhibitor that can be used in any methods presented here is any of Compounds Ga-Gc: 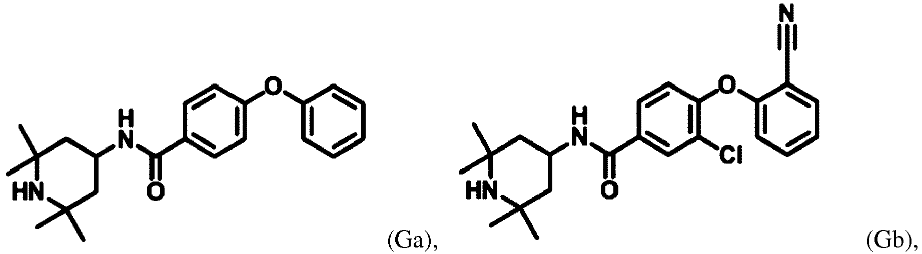 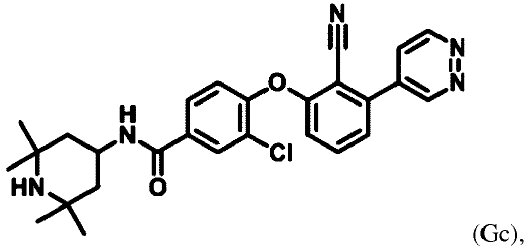 or a stereoisomer, pharmaceutically acceptable salt or solvate thereof.

In certain embodiments, an EZH2 inhibitor that can be used in any methods presented here is CPI-1205 or GSK343.

Additional suitable EZH2 inhibitors will be apparent to those skilled in the art. In some embodiments of the strategies, treatment modalities, methods, combinations, and compositions provided herein, the EZH2 inhibitor is an EZH2 inhibitor described in US 8,536,179 (describing GSK-126 among other compounds and corresponding to WO 2011/140324), the entire contents of each of which are incorporated herein by reference.

In some embodiments of the strategies, treatment modalities, methods, combinations, and compositions provided herein, the EZH2 inhibitor is an EZH2 inhibitor described in PCT/US2014/015706, published as WO 2014/124418, in PCT/US2013/025639, published as WO 2013/120104, and in US 14/839,273, published as US 2015/0368229, the entire contents of each of which are incorporated herein by reference.

In one embodiment, the compound (e.g., an EZH2 inhibitor) disclosed herein is the compound itself, i.e., the free base or "naked" molecule. In another embodiment, the compound is a salt thereof, *e.g.,* a mono-HCl or tri-HCl salt, mono-HBr or tri-HBr salt of the naked molecule.

Compounds disclosed herein that contain nitrogens can be converted to N-oxides by treatment with an oxidizing agent (*e.g.,* 3-chloroperoxybenzoic acid (*m*CPBA) and/or hydrogen peroxides) to afford other compounds suitable for any methods disclosed herein. Thus, all shown and claimed nitrogen-containing compounds are considered, when allowed by valency and structure, to include both the compound as shown and its N-oxide derivative (which can be designated as N→O and N⁺-O⁻). Furthermore, in other instances, the nitrogens in the compounds disclosed herein can be converted to N-hydroxy or N-alkoxy compounds. For example, N-hydroxy compounds can be prepared by oxidation of the parent amine by an oxidizing agent such as m-CPBA. All shown and claimed nitrogen-containing compounds are also considered, when allowed by valency and structure, to cover both the compound as shown and its N-hydroxy (*i.e.,* N-OH) and N-alkoxy (*i.e.,* N-OR, wherein R is substituted or unsubstituted C₁-C₆ alkyl, C₁-C₆ alkenyl, C₁-C₆ alkynyl, 3-14-membered carbocycle or 3-14-membered heterocycle) derivatives.

"Isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

A carbon atom bonded to four nonidentical substituents is termed a "chiral center."

"Chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

"Geometric isomer" means the diastereomers that owe their existence to hindered rotation about double bonds or a cycloalkyl linker (*e.g.,* 1,3-cylcobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds disclosed herein may be depicted as different chiral isomers or geometric isomers. It should also be understood that when compounds have chiral isomeric or geometric isomeric forms, all isomeric forms are intended to be included in the scope of the disclosure, and the naming of the compounds does not exclude any isomeric forms.

Furthermore, the structures and other compounds discussed in this disclosure include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertable by tautomerizations is called tautomerism.

The compounds disclosed herein include the compounds themselves, as well as their salts and their solvates, if applicable. A salt, for example, can be formed between an anion and a positively charged group (*e.g.,* amino) on an aryl- or heteroaryl-substituted benzene compound. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (*e.g.,* trifluoroacetate). The term "pharmaceutically acceptable anion" refers to an anion suitable for forming a pharmaceutically acceptable salt. Likewise, a salt can also be formed between a cation and a negatively charged group (*e.g.,* carboxylate) on an aryl- or heteroaryl-substituted benzene compound. Suitable cations include sodium ion, potassium ion, magnesium ion, calcium ion, and an ammonium cation such as tetramethylammonium ion. The aryl- or heteroaryl-substituted benzene compounds also include those salts containing quaternary nitrogen atoms. In the salt form, it is understood that the ratio of the compound to the cation or anion of the salt can be 1:1, or any ration other than 1:1, *e.g.,* 3:1, 2:1, 1:2, or 1:3.

Additionally, the compounds disclosed herein, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative" refers to compounds that have a common core structure, and are substituted with various groups as described herein. For example, all of the compounds represented by Formula (I) are aryl- or heteroaryl-substituted benzene compounds, and have Formula (I) as a common core.

The present disclosure is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

As used herein, "treating" or "treat" describes the management and care of a patient for the purpose of combating a disease, condition, or disorder and includes the administration of a compound disclosed herein, or a pharmaceutically acceptable salt or solvate thereof, to alleviate the symptoms or complications of a disease, condition or disorder, or to eliminate the disease, condition or disorder.

A composition disclosed herein, or a pharmaceutically acceptable salt or solvate thereof, can also be used to prevent a disease, condition or disorder. As used herein, "preventing" or "prevent" describes reducing or eliminating the onset of the symptoms or complications of the disease, condition or disorder.

As used herein, the term "alleviate" is meant to describe a process by which the severity of a sign or symptom of a disorder is decreased. Importantly, a sign or symptom can be alleviated without being eliminated. In a preferred embodiment, the administration of pharmaceutical compositions disclosed herein leads to the elimination of a sign or symptom, however, elimination is not required. Effective dosages are expected to decrease the severity of a sign or symptom. For instance, a sign or symptom of a disorder such as cancer, which can occur in multiple locations, is alleviated if the severity of the cancer is decreased within at least one of multiple locations.

As used herein, the term "severity" is meant to refer to the stage or the potential for progression of a disease. In the context of cancer, severity typically refers to the potential of a cancer to transform from a precancerous, or benign, state into a malignant state. Alternatively, or in addition, severity may describe a cancer stage, for example, according to the TNM system (accepted by the International Union Against Cancer (UICC) and the American Joint Committee on Cancer (AJCC)) or by other art-recognized methods. Cancer stage refers to the extent or severity of the cancer, based on factors such as the location of the primary tumor, tumor size, number of tumors, and lymph node involvement (spread of cancer into lymph nodes). Alternatively, or in addition, severity is meant to describe the tumor grade by art-recognized methods (see, National Cancer Institute, www.cancer.gov). Tumor grade is a system used to classify cancer cells in terms of how abnormal they look under a microscope and how quickly the tumor is likely to grow and spread. Many factors are considered when determining tumor grade, including the structure and growth pattern of the cells. The specific factors used to determine tumor grade vary with each type of cancer. Severity also describes a histologic grade, also called differentiation, which refers to how much the tumor cells resemble normal cells of the same tissue type (see, National Cancer Institute, www.cancer.gov). Furthermore, severity describes a nuclear grade, which refers to the size and shape of the nucleus in tumor cells and the percentage of tumor cells that are dividing (see, National Cancer Institute, www.cancer.gov).

In another aspect, severity describes the degree to which a tumor has secreted growth factors, degraded the extracellular matrix, become vascularized, lost adhesion to juxtaposed tissues, or metastasized. Moreover, severity describes the number of locations to which a primary tumor has metastasized. Finally, severity includes the difficulty of treating tumors of varying types and locations. For example, inoperable tumors, those cancers which have greater access to multiple body systems (hematological and immunological tumors), and those which are the most resistant to traditional treatments are considered most severe. In these situations, prolonging the life expectancy of the subject and/or reducing pain, decreasing the proportion of cancerous cells or restricting cells to one system, and improving cancer stage/tumor grade/histological grade/nuclear grade are considered alleviating a sign or symptom of the cancer.

As used herein the term "symptom" is defined as an indication of disease, illness, injury, or that something is not right in the body. Symptoms are felt or noticed by the individual experiencing the symptom, but may not easily be noticed by others. Others are defined as non-health-care professionals.

As used herein the term "sign" is also defined as an indication that something is not right in the body. But signs are defined as things that can be seen by a doctor, nurse, or other health care professional.

Cancer is a group of diseases that may cause almost any sign or symptom. The signs and symptoms will depend on where the cancer is, the size of the cancer, and how much it affects the nearby organs or structures. If a cancer spreads (metastasizes), then symptoms may appear in different parts of the body.

Treating cancer can result in a reduction in size of a tumor. A reduction in size of a tumor may also be referred to as "tumor regression". Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. The size of a tumor may be measured as a diameter of the tumor.

Treating cancer can result in a reduction in tumor volume. Preferably, after treatment, tumor volume is reduced by 5% or greater relative to its size prior to treatment; more preferably, tumor volume is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Tumor volume may be measured by any reproducible means of measurement.

Treating cancer results in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. The number of tumors may be measured by counting tumors visible to the naked eye or at a specified magnification. Preferably, the specified magnification is 2x, 3x, 4x, 5x, 10x, or 50x.

Treating cancer can result in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. The number of metastatic lesions may be measured by counting metastatic lesions visible to the naked eye or at a specified magnification. Preferably, the specified magnification is 2x, 3x, 4x, 5x, 10x, or 50x.

Treating cancer can result in an increase in average survival time of a population of treated subjects in comparison to a population receiving carrier alone. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer can result in an increase in average survival time of a population of treated subjects in comparison to a population of untreated subjects. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer can result in increase in average survival time of a population of treated subjects in comparison to a population receiving monotherapy with a drug that is not a compound disclosed herein, or a pharmaceutically acceptable salt, solvate, analog or derivative thereof. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer can result in a decrease in the mortality rate of a population of treated subjects in comparison to a population receiving carrier alone. Treating cancer can result in a decrease in the mortality rate of a population of treated subjects in comparison to an untreated population. Treating cancer can result in a decrease in the mortality rate of a population of treated subjects in comparison to a population receiving monotherapy with a drug that is not a compound disclosed herein, or a pharmaceutically acceptable salt, solvate, analog or derivative thereof. Preferably, the mortality rate is decreased by more than 2%; more preferably, by more than 5%; more preferably, by more than 10%; and most preferably, by more than 25%. A decrease in the mortality rate of a population of treated subjects may be measured by any reproducible means. A decrease in the mortality rate of a population may be measured, for example, by calculating for a population the average number of disease-related deaths per unit time following initiation of treatment with an active compound. A decrease in the mortality rate of a population may also be measured, for example, by calculating for a population the average number of disease-related deaths per unit time following completion of a first round of treatment with an active compound.

Treating cancer can result in a decrease in tumor growth rate. Preferably, after treatment, tumor growth rate is reduced by at least 5% relative to number prior to treatment; more preferably, tumor growth rate is reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Tumor growth rate may be measured by any reproducible means of measurement. Tumor growth rate can be measured according to a change in tumor diameter per unit time.

Treating cancer can result in a decrease in tumor regrowth. Preferably, after treatment, tumor regrowth is less than 5%; more preferably, tumor regrowth is less than 10%; more preferably, less than 20%; more preferably, less than 30%; more preferably, less than 40%; more preferably, less than 50%; even more preferably, less than 50%; and most preferably, less than 75%. Tumor regrowth may be measured by any reproducible means of measurement. Tumor regrowth is measured, for example, by measuring an increase in the diameter of a tumor after a prior tumor shrinkage that followed treatment. A decrease in tumor regrowth is indicated by failure of tumors to reoccur after treatment has stopped.

Treating or preventing a cell proliferative disorder can result in a reduction in the rate of cellular proliferation. Preferably, after treatment, the rate of cellular proliferation is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The rate of cellular proliferation may be measured by any reproducible means of measurement. The rate of cellular proliferation is measured, for example, by measuring the number of dividing cells in a tissue sample per unit time.

Treating or preventing a cell proliferative disorder can result in a reduction in the proportion of proliferating cells. Preferably, after treatment, the proportion of proliferating cells is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The proportion of proliferating cells may be measured by any reproducible means of measurement. Preferably, the proportion of proliferating cells is measured, for example, by quantifying the number of dividing cells relative to the number of nondividing cells in a tissue sample. The proportion of proliferating cells can be equivalent to the mitotic index.

Treating or preventing a cell proliferative disorder can result in a decrease in size of an area or zone of cellular proliferation. Preferably, after treatment, size of an area or zone of cellular proliferation is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Size of an area or zone of cellular proliferation may be measured by any reproducible means of measurement. The size of an area or zone of cellular proliferation may be measured as a diameter or width of an area or zone of cellular proliferation.

Treating or preventing a cell proliferative disorder can result in a decrease in the number or proportion of cells having an abnormal appearance or morphology. Preferably, after treatment, the number of cells having an abnormal morphology is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. An abnormal cellular appearance or morphology may be measured by any reproducible means of measurement. An abnormal cellular morphology can be measured by microscopy, *e.g.,* using an inverted tissue culture microscope. An abnormal cellular morphology can take the form of nuclear pleiomorphism.

As used herein, the term "selectively" means tending to occur at a higher frequency in one population than in another population. The compared populations can be cell populations. Preferably, a compound disclosed herein, or a pharmaceutically acceptable salt or solvate thereof, acts selectively on a cancer or precancerous cell but not on a normal cell. Preferably, a compound disclosed herein, or a pharmaceutically acceptable salt or solvate thereof, acts selectively to modulate one molecular target (*e.g.,* a target protein methyltransferase) but does not significantly modulate another molecular target (*e.g.,* a non-target protein methyltransferase). The disclosure also provides a method for selectively inhibiting the activity of an enzyme, such as a protein methyltransferase. Preferably, an event occurs selectively in population A relative to population B if it occurs greater than two times more frequently in population A as compared to population B. An event occurs selectively if it occurs greater than five times more frequently in population A. An event occurs selectively if it occurs greater than ten times more frequently in population A; more preferably, greater than fifty times; even more preferably, greater than 100 times; and most preferably, greater than 1000 times more frequently in population A as compared to population B. For example, cell death would be said to occur selectively in cancer cells if it occurred greater than twice as frequently in cancer cells as compared to normal cells.

A composition disclosed herein, *e.g.,* a composition comprising any compound disclosed herein or pharmaceutically acceptable salt thereof, can modulate the activity of a molecular target (*e.g.,* a target protein methyltransferase). Modulating refers to stimulating or inhibiting an activity of a molecular target. Preferably, a compound disclosed herein, or a pharmaceutically acceptable salt or solvate thereof, modulates the activity of a molecular target if it stimulates or inhibits the activity of the molecular target by at least 2-fold relative to the activity of the molecular target under the same conditions but lacking only the presence of said compound. More preferably, a compound disclosed herein, or a pharmaceutically acceptable salt or solvate thereof, modulates the activity of a molecular target if it stimulates or inhibits the activity of the molecular target by at least 5-fold, at least 10-fold, at least 20-fold, at least 50-fold, at least 100-fold relative to the activity of the molecular target under the same conditions but lacking only the presence of said compound. The activity of a molecular target may be measured by any reproducible means. The activity of a molecular target may be measured *in vitro* or *in vivo.* For example, the activity of a molecular target may be measured *in vitro* by an enzymatic activity assay or a DNA binding assay, or the activity of a molecular target may be measured *in vivo* by assaying for expression of a reporter gene.

A composition disclosed herein does not significantly modulate the activity of a molecular target if the addition of the compound does not stimulate or inhibit the activity of the molecular target by greater than 10% relative to the activity of the molecular target under the same conditions but lacking only the presence of said compound.

Preferably, a compound disclosed herein, or a pharmaceutically acceptable salt or solvate thereof, demonstrates a minimum of a fourfold differential, preferably a tenfold differential, more preferably a fifty fold differential, in the dosage required to achieve a biological effect. Preferably, a compound disclosed herein, or a pharmaceutically acceptable salt or solvate thereof, demonstrates this differential across the range of inhibition, and the differential is exemplified at the IC₅₀, *i.e.,* a 50% inhibition, for a molecular target of interest.

As used herein, the term "flow cytometry" refers to a biophysical technology employed in cell counting, cell sorting, characterization of cells, quantification of cells, biomarker detection, and protein engineering, characterized by suspending cells of interest in a stream of fluid and passing them by a detection apparatus. In certain embodiments, flow cytometry may be used in the context of Fluorescence-Activated Cell Sorting (FACS), a laser based assay used for assessing the presence or absence of a fluorescent compound attached to a cell, optionally performed in a quantitative manner, such that the number of cells having the particular fluorescent compound attached can be quantified from a mixed cellular sample. In certain embodiments, flow cytometry may be used in the context of Magnetic-Activated Cell Sorting (MACS), a method for assessing the presence or absence of a magnetic or paramagnetic compound attached to a cell, optionally performed in a quantitative manner, such that the number of cells having the particular magnetic or paramagnetic compound attached can be quantified from a mixed cellular sample.

The term "antibody" is meant to include polyclonal antibodies, monoclonal antibodies (MAbs), chimeric antibodies, anti-idiotypic (anti-Id) antibodies to antibodies that can be labeled in soluble or bound form, and humanized antibodies as well as fragments thereof provided by any known technique, such as, but not limited to enzymatic cleavage, peptide synthesis or recombinant techniques. The terms "antibody" and "immunoglobulin" are used interchangeably.

An antibody is said to be "capable of binding" a molecule if it is capable of specifically reacting with the molecule to thereby bind the molecule to the antibody. The term "epitope" is meant to refer to that portion of any molecule capable of being bound by an antibody which can also be recognized by that antibody. Epitopes or "antigenic determinants" usually consist of chemically active surface groupings of molecules such as amino acids or sugar side chains and have specific three dimensional structural characteristics as well as specific charge characteristics.

An "antigen" is a molecule or a portion of a molecule capable of being bound by an antibody which is additionally capable of inducing an animal to produce antibody capable of binding to an epitope of that antigen. An antigen may have one or more than one epitope. An epitope is a part of an antigen recognized by an antibody. The specific reaction referred to above is meant to indicate that the antigen will react, in a highly selective manner, with its corresponding antibody and not with the multitude of other antibodies which may be evoked by other antigens.

Administering a composition disclosed herein to a cell or a subject in need thereof can result in modulation (e.g., stimulation or inhibition) of an activity of a protein methyltransferase of interest.

Administering a compound disclosed herein, *e.g.,* a composition comprising any compound disclosed herein or pharmaceutically acceptable salt thereof, and one or more other therapeutic agents, such as prednisone, to a cell or a subject in need thereof results in modulation (*e.g.,* stimulation or inhibition) of an activity of an intracellular target (*e.g.,* substrate). Several intracellular targets can be modulated with the compounds disclosed herein, including, but not limited to, protein methyltrasferase.

Activating refers to placing a composition of matter (*e.g.,* protein or nucleic acid) in a state suitable for carrying out a desired biological function. A composition of matter capable of being activated also has an unactivated state. An activated composition of matter may have an inhibitory or stimulatory biological function, or both.

An "activating mutation" is a mutation that results in increased and/or constitutive biological activity. For example, an activating mutation results in constitutive biological activity in the absence of physiological activators. As used herein, the term "activating mutation" includes a "gain-of-function" mutation.

A "linear epitope" is an epitope that is recognized by an antibody according to the epitope's linear sequence of amino acids (primary structure). A linear epitope may also be a conformational epitope.

A "conformational epitope" is an epitope recognized by an antibody according to the epitope's three-dimensional shape.

A "discontinuous epitope" is an epitope recognized by an antibody according to a three-dimensional shape and/or non-adjacent residues or portions thereof. A "non-linear" epitope of the methods of the disclosure may comprise, consist essentially of, or consist of a discontinuous epitope.

A "conjugated antibody" is an antibody (monoclonal or polyclonal) that is linked to another compound. For example, a conjugated antibody can be linked to a fluorescent or chromogenic label.

Elevation refers to an increase in a desired biological activity of a composition of matter (*e.g.,* a protein or a nucleic acid). Elevation may occur through an increase in concentration of a composition of matter.

Treating cancer or a cell proliferative disorder can result in cell death, and preferably, cell death results in a decrease of at least 10% in number of cells in a population. More preferably, cell death means a decrease of at least 20%; more preferably, a decrease of at least 30%; more preferably, a decrease of at least 40%; more preferably, a decrease of at least 50%; most preferably, a decrease of at least 75%. Number of cells in a population may be measured by any reproducible means. A number of cells in a population can be measured by fluorescence activated cell sorting (FACS), immunofluorescence microscopy and light microscopy. Methods of measuring cell death are as shown in Li et al., Proc Natl Acad Sci U S A. 100(5): 2674-8, 2003. In an aspect, cell death occurs by apoptosis.

Preferably, an effective amount of a composition disclosed herein, or a pharmaceutically acceptable salt or solvate thereof, is not significantly cytotoxic to normal cells. A therapeutically effective amount of a compound is not significantly cytotoxic to normal cells if administration of the compound in a therapeutically effective amount does not induce cell death in greater than 10% of normal cells. A therapeutically effective amount of a compound does not significantly affect the viability of normal cells if administration of the compound in a therapeutically effective amount does not induce cell death in greater than 10% of normal cells. In an aspect, cell death occurs by apoptosis.

Contacting a cell with an EZH2 inhibitor or composition thereof disclosed herein, or a pharmaceutically acceptable salt or solvate thereof, can induce or activate cell death selectively in cancer cells. Administering to a subject in need thereof a compound disclosed herein, or a pharmaceutically acceptable salt or solvate thereof, can induce or activate cell death selectively in cancer cells. Contacting a cell with a composition disclosed herein, or a pharmaceutically acceptable salt or solvate thereof, can induce cell death selectively in one or more cells affected by a cell proliferative disorder. Preferably, administering to a subject in need thereof a composition disclosed herein, or a pharmaceutically acceptable salt or solvate thereof, induces cell death selectively in one or more cells affected by a cell proliferative disorder.

The present disclosure relates to a method of treating or preventing cancer by administering a composition disclosed herein, or a pharmaceutically acceptable salt or solvate thereof, to a subject in need thereof, where administration of the composition disclosed herein, or a pharmaceutically acceptable salt or solvate thereof, results in one or more of the following: prevention of cancer cell proliferation by accumulation of cells in one or more phases of the cell cycle (*e.g.* G1, G1/S, G2/M), or induction of cell senescence, or promotion of tumor cell differentiation; promotion of cell death in cancer cells via cytotoxicity, necrosis or apoptosis, without a significant amount of cell death in normal cells, antitumor activity in animals with a therapeutic index of at least 2. As used herein, "therapeutic index" is the maximum tolerated dose divided by the efficacious dose.

One skilled in the art may refer to general reference texts for detailed descriptions of known techniques discussed herein or equivalent techniques. These texts include Ausubel et al., Current Protocols in Molecular Biology, John Wiley and Sons, Inc. (2005); Sambrook et al., Molecular Cloning, A Laboratory Manual (3rd edition), Cold Spring Harbor Press, Cold Spring Harbor, New York (2000); Coligan et al., Current Protocols in Immunology, John Wiley & Sons, N.Y.; Enna et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, PA, 18th edition (1990). These texts can, of course, also be referred to in making or using an aspect of the disclosure.

In order that the invention disclosed herein may be more efficiently understood, examples are provided below. It should be understood that these examples are for illustrative purposes only and are not to be construed as limiting the disclosure in any manner.

### Example 1: Detection of Epigenetic Modification by Flow Cytometry

A protocol for the detection of H3K27me3 by flow cytometry methodology is presented below.

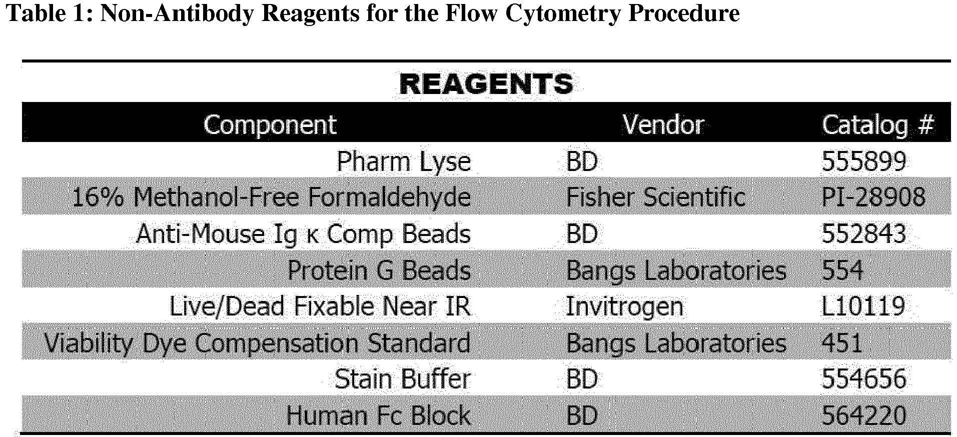

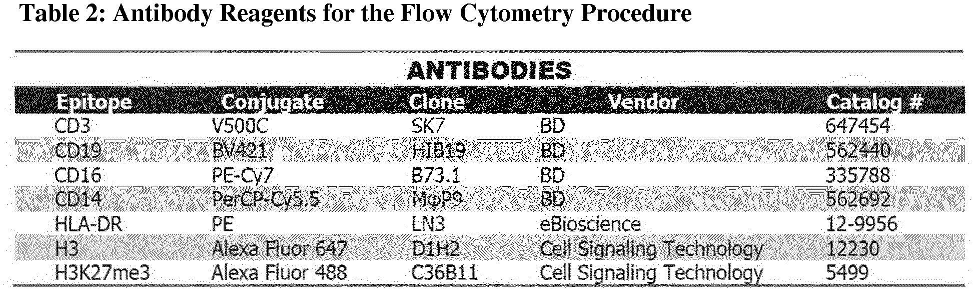

### Protocol for Red Blood Cell Lysis and Viability Staining

1. Aliquot 2ml whole blood to 50ml conical tube. (Note: Do not attempt to scale up beyond 2ml for a single 50mL tube as RBC lysis will be incomplete. However, pellets from 2 50mL tubes may be combined into a single 5mL tube at step #7.)
2. Add 20ml of 1X BD Pharm Lyse and vortex gently.
3. Incubate 15 minutes at room temperature on a rocker and protected from light.
4. Add 15mL PBS and centrifuge at 300 x g for 5 minutes.
5. Aspirate fluid with small diameter tip (*e.g.,* 20µl Rainin pipette tip) leaving 1-2ml fluid over the pellet. Take care not to aspirate any cells.
6. Use a 1mL pipette to resuspend the pellet in remaining fluid.
7. Transfer the suspension to a 5mL round bottom FACS tube.
8. Add PBS to a volume of 4mL and centrifuge at 300 x g for 5 minutes.
9. Aspirate PBS leaving ∼500µL residual volume.
10. Wash cells using the following 2x2 resuspension technique: add 2mL PBS (not staining solution containing BSA or azide), use a 1mL pipette to dissociate the pellet and resuspend the cells, add a further 2mL PBS. (Note: Do not add the full 4mL and attempt to resuspend by vortex or inversion. Resuspension will be incomplete.)
11. Centrifuge at 300 x g for 5 minutes.
12. Aspirate PBS leaving 500µL residual volume and resuspend the pellet in 4mL PBS as detailed above (2x2 technique).
13. Add 4uL of reconstituted Invitrogen LIVE/DEAD Fixable Near-IR Dead Cell Stain Kit.
14. Incubate 30 minutes at room temperature on a rocker and protected from light.
15. Centrifuge at 300 x g for 5 minutes and resuspend in 4mL PBS via 2x2 technique.
16. Centrifuge as above, aspirate as above and resuspend in 2mL PBS with 1mL pipette.
17. Add another 1mL to the tube (3mL total).
18. Add 1mL of 16% methanol-free formaldehyde to the tube (4% final concentration).
19. Incubate 15 minutes at room temperature on a rocker and protected from light.
20. Centrifuge, aspirate and resuspend in 4mL as above.
21. Centrifuge and aspirate as above.
22. Add 2mL of 0.1% Triton X-100, resuspend with 1mL pipette, add a further 2mL of 0.1% Triton X-100.
23. Incubate 15 minutes at room temperature on a rocker and protected from light.
24. Centrifuge, aspirate and resuspend in 4mL as above.
25. Centrifuge and aspirate as above.
26. Resuspend in 2mL of BD Stain Buffer.
27. Count both viable and non-viable cells (permeabilized cells will take up trypan blue).
28. Resuspend in appropriate volume for 3-5x10⁶ cells/mL³. (Note: Expected yield for a 2mL aliquot of whole blood should be >8x10⁶ cells).
29. Proceed to staining or store at 4°C, protected from light overnight.

### Protocol for Antibody Staining

1. Aliquot 100µl of cell suspension per test well (96 well plate).
2. Add 5uL BD Human Fc Block to each test well and mix by pipetting up and down 5 times.
3. Incubate 10min at room temperature with mild shaking.
4. Add anti-H3-Alexa Fluor 647 at 1:75 to each test well and mix by pipetting up and down 5 times.
5. Incubate at room temperature for 45 minutes with mild shaking.
6. Add remaining antibodies at the dilutions listed in Table 3.
7. Create compensation controls. (Note: Voltages should be standardized for the staining panel and verified against know standards each day (for BD instruments this means creating application settings for the panel and calibrating with CST beads prior to use). All surface marker antibodies are mouse κ antibodies and can be bound to BD Comp Beads. H3 and H3K27me3 antibodies are rabbit IgG and can be bound to Bangs Labs Protein G beads. The Bangs Labs Viability Dye Compensation Standard should be used for Live/Dead compensation.)
8. Mix all wells by pipetting up and down 5 times.
9. Incubate at room temperature for 1 hour with mild shaking.
10. Centrifuge plates at 300 x g for 5 minutes.
11. Decant plate using fast wrist motion into sink followed by rapid gentle contact with lab tissue paper (do not delay and do not invert in between motions).
12. Add 200µL BD Stain Buffer to test & compensation wells and mix by pipetting up and down 5 times.
13. Centrifuge plates at 300 x g for 5 minutes.
14. Decant as above.
15. Add 200µL BD Stain Buffer and proceed to analysis.

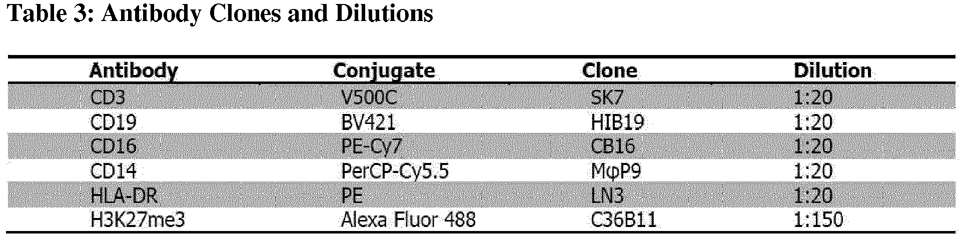

### Example 2: Sample Preparation-Permeabilization Methods Comparison

Various permeabilization methods were compared to assess the effect of permeabilization on the preservation of surface epitope detection in a blood sample. The permeabilization technique ultimately chosen should allow for accurate population discrimination based on surface marker identification. Figure 1 (*i.e.* Figure 1A and 1B) depicts the results of the preservation of the surface epitopes HLA-DR (LN3), CD14, CD3, CD14, CD16 and CD19 when a mixed population of blood cells were contacted with 0.1% triton X-100 solution, a 70% methanol solution, or PBS alone (no permeabilization solution). After the cells were incubated in these conditions, the cells were washed and incubated with a cocktail of antibodies directed at surface epitopes (*e.g.,* HLA-DR (LN3), CD14, CD3, CD14, CD16 and CD19). The binding of the antibodies to the cells was assessed by flow cytometry. The data clearly indicate that permeabilization with 70% methanol resulted in a large decrease in the ability to detect surface antigens. Figure 1 demonstrates that incubation with 70% methanol resulted in a near complete loss of CD19 and CD14 epitope detection. In contrast, permeabilization with a 0.1% triton X-100 solution allowed for the detection of all of the surface epitopes tested.

Another series of experiments was directed at the comparison of permeabilization of a blood sample with 50% methanol in comparison to a no methanol condition. These data are presented in Figure 1B. The data demonstrate that detection of the surface marker CD16 is drastically reduced in comparison to the no methanol condition.

Collectively, these data demonstrate that permeabilization with methanol does not allow for the preservation of a range of surface epitopes in comparison to permeabilization with 0.1% triton X-100.

### Example 3: Selection of Antibodies to Detect Surface Markers

A series of antibody clones were tested using the flow cytometry protocol of Example 1. Representative findings from these experiments are presented in Figure 2 (*i.e.* Figures 2A and 2B). The data indicate that different antibody clones targeted to an identical epitope have varied tolerance to sample preparation techniques, had differential behavior based on isotype, demonstrated differences in regard to non-specific binding, and had variations in optimal titration concentration. *See* Figures 2A and 2C. Similar findings were also found in the selection of fluorophores. *See* Figure 2B. In particular, different fluorophores demonstrated variations in relative brightness, instrument compatibility, dye stability, and background fluorescence levels. Flow cytometry analysis of antibodies and corresponding fluorophores allowed for the identification of the antibodies and dilutions presented in Tables 2 and 3.

### Example 4: Assessment of Variations in H3K27 Methylation Patterns in a Cell Sample

Flow cytometry was used to ascertain the H3K27me3 status in the OCI-LY19 cell line. As a normalization control for this flow cytometry study, the total amount of Histone 3 (H3) was assessed in the OCI-LY19 cell line. The data are presented in Figure 3 (*i.e.* Figures 3A, 3B, and 3C). The flow cytometry plot in Figure 3A shows that the addition of EPZ007210 to the OCI-LY19 cell line results in the reduction of the H3K27me3 signal in comparison to the no treatment, naive control. Figure 3B shows that addition of EPZ007210 to the OCI-LY19 cell line does not affect the amount of total H3 detected. This indicates that the use of an antibody that binds H3 can be used as a normalization control. Figure 3C is a chart that depicts the mean fluorescence intensity observed by flow cytometry for the presence of H3K27me3 and total H3 following contacting the OCI-LY19 cell line with EPZ007210.

As a proof of concept, a dose response experiment was conducted using EPZ007210 with the WSU cell line (EZH2 mutant) and the OCI-LY19 (EZH2) cell line. *See* Figure 4A. The amounts of EPZ007210 contacted with the cell lines varied per condition as follows: 0nM, 03nM, 1nM, 4nM, 12nM, 37nM, 111nM, 333nM and 1000nM. The data indicate that the H3K27me3 signal was reduced with increasingly higher amounts of EPZ007210 exposure in both the WSU cell line (EZH2 mutant) and the OCI-LY19 (EZH2) cell line. The amount of H3K27me2 remained stable in the WSU cell line (EZH2 mutant), while there was a reduction in the H3K27me2 signal with increasingly higher amounts of EPZ007210 incubated with the OCI-LY19 cell line. Importantly, there was no change in the total amounts of H3 signal in either cell line following exposure to the varying concentrations of EPZ007210. The flow cytometry observations were confirmed by Western Blot assays. *See* Figure 4B.

Collectively, these data indicate that in one embodiment the use of flow cytometry can be used to assess changes in H3K27 methylation status following *in vitro* exposure of cells to an EZH2 inhibitor using the protocol detailed in Example 1.

### Example 5: Development of a Flow Cytometry Assay for the Detection of Surface and Nuclear Antigens

The simultaneous detection of surface antigens and H3K27 methylation status was determined by flow cytometry. For this series of experiments, various antibody panels were used to ascertain which panel, if any, provided for strong signal detection in various conditions. For each of these panels, the background signal, clone performance and titrations were individually optimized. Another important consideration in the development of the panels was to pair fluorophores with epitopes based on brightness. Table 4 below indicates select panels that were assayed. Tables 5A-5C have listings of markers and the cell types that these markers identify.

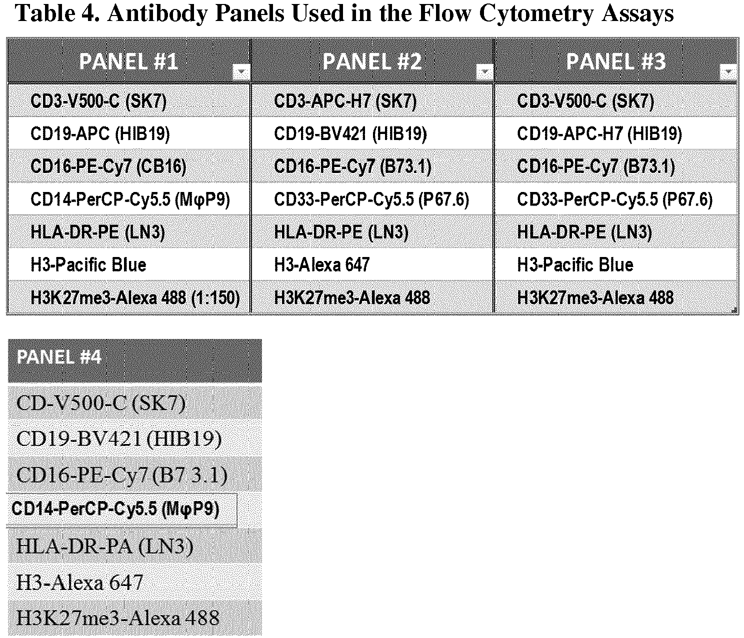

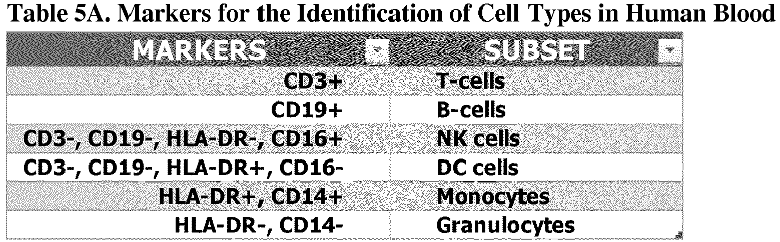

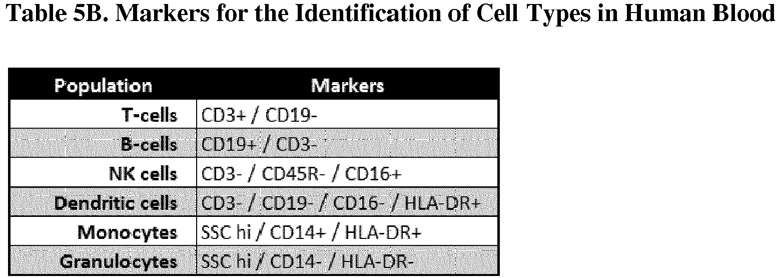

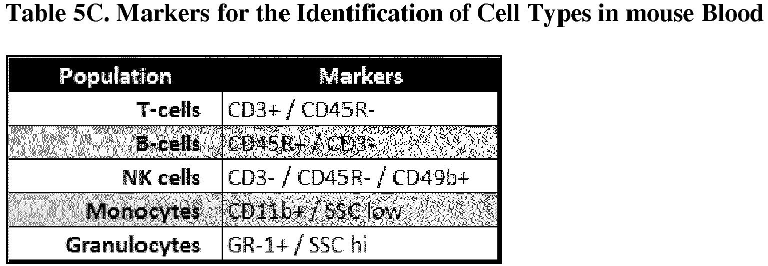

The simultaneous detection of surface epitopes and nuclear antigens in a blood sample by flow cytometry is shown in Figures 5A-5D. Figures 5A and 5B show the detection of various antigens corresponding to surface epitopes of several blood cells (*e.g.,* B-cells, T cells, monocytes, and granulocytes) by flow cytometry. Figure 5C shows representative flow cytometry scatter plots and histogram plots using antibodies from panel 1. These scatter plots illustrate that the HK27me3 status of specific kinds of cells can be identified through the use of flow cytometry according to the protocol described in example 1 and through the use of antibodies listed in Table 4. Figure 5D shows the inclusion of an antibody that binds histone 3 (H3) that is used for the normalization of the signal obtained from the H3K27me3 antibody. These data indicate variations in the amounts of H3K27me3 found in various kinds of cells obtained from a blood sample.

The reproducibility of antibody detection of surface epitopes and H3K27me3 was ascertained by contacting blood samples obtained from one donor with various antibody panels of Table 4. *See* Figure 6A. These data indicate that there is a strong correlation in the H3K27me3 signal obtained with a single sample stained with two different antibody panels. Inter-donor reproducibility was also assessed. For these assays, blood samples from two separate donors were processed with a panel of antibodies from Table 4. The data indicate that there are subtle differences in the amounts of H3K27me3 between donor samples; however, the overall pattern of signal in the peripheral blood populations is conserved. *See* Figure 6B.

A comparison between blood cell type and H3K27me3 status was also assessed. The data from these assays revealed that there are differences in the amounts of H3K27me3 found on various kinds of blood cell types. *See* Figure 7. For example, B-cells have high H3K27me3 presence, but only make up 14% of peripheral blood mononuclear cells (PBMCs) and 3% of all peripheral white blood cells (WBCs). Importantly, the modulation of signal in cell types with high levels of methylation and low overall representation is difficult to detect by Western assays or with ELISA assays. However, these differences can be ascertained through the use of the flow cytometry assays described herein.

### Example 6: Assessment by Flow Cytometry of In Vivo Dose Response Following Administration of EZH2 inhibitor EPZ-6438

An *in vivo* rodent dose response study was performed to ascertain the effect of administration of the EZH2 inhibitor EPZ-6438. For this study, the following dosage conditions were established: vehicle only, 125 mg/kg, 250 mg/kg, and 500 mg/kg. The EPZ-6438 administration frequency was twice per day, every 12 hours by mouth for a total of seven days. Following this period, blood was collected from the rodent and processed as described in Example 1. For this study antibody panel 1 was used. The data indicate that there is good signal and population separation on all fluorescence channels, and that the labeling intensity was consistent between tubes. *See* Figure 8. Also noted in this study was that a small amount of clotting of the blood resulted in decreased cell yield. The data obtained from these studies is summarized in Figure 9. The results indicate that only the monocytes and natural killer (NK) cells demonstrate a dose dependent methyl mark reduction following administration of EPZ-6438. Monocytes and NK cells comprise 9.2% and 6.5%, respectively, of leukocytes assayed. Reduction in the amounts of H3K27me3 in the monocyte and NK cell population in relation to total methyl mark reduction is 10% given the combined amounts of these cells found in peripheral blood. The contribution of discrete cellular populations on the bulk H3K27me3 signal is shown in Figure 10.

The H3K27me3 data obtained by flow cytometry was compared to ELISA assays performed with bulk blood cell populations. *See* Figure 11. As shown in Figure 10, monocyte response is far more robust than that from the bulk population. This comparison of assays reveals that the flow cytometry assay provides a greater sensitivity to changes in methyl mark by examining the most responsive cell populations (*e.g.,* monocytes and NK cells). The flow cytometry assays further support that the dose responsive result is possible from a 100-300µL whole blood sample.

### Example 7: Chromatin flow cytometry based assessment of H3K27me3 pharmacodynamics in blood from Diffuse Large B-Cell Lymphoma (DLBCL) and Follicular Lymphoma (FL) patients following exposure to the EZH2 inhibitor Tazemetostat reveals disparate response profiles in specific PBMC subpopulations.

Monitoring the pharmacodynamics (PD) of histone methylation in blood has routinely relied on analysis of bulk peripheral blood mononuclear cell (PBMC) lysates or purified histones. These methods demonstrated minimal change in H3K27me3 levels using blood samples collected in the tazemetostat phase 1 study at all doses tested (100 - 1600 mg dosed twice daily (BID)). In contrast significant reductions (up to 50%) in H3K27me3 levels were observed in post dose skin biopsies collected on the phase 1 study at doses ranging from 400 - 1600 mg BID. Given the apparently different PD responses to tazemetostat in two surrogate tissues the possibility that PBMC subsets may respond differentially to tazemetostat exposure was explored by developing a flow cytometry based epigenetic assay to examine H3K27me3 levels in PBMC subsets.

Peripheral blood from DLBCL or FL patients enrolled onto the ongoing tazemetostat phase 2 NHL trial were collected pre-dose (C1D1) and at two post dose time points, cycle 1 day 15 (C1D15) and cycle 2 day 1 (C2D1, 30 days post-baseline). Flow cytometry based immunophenotyping of B-cell, T-cell, granulocyte and monocyte populations, along with fluorescent staining of H3K27me3 and total histone H3 (H3) antibodies, allowed for quantification of normalized H3K27me3 levels in PBMC subsets.

**Table 6: Mean +/- Standard Deviation (SD) (displayed as [mean/SD]) of H3K27me3 level (normalized to H3) for each PBMC subset detected:**

| **Cell Type** | **C1D1 n = 36** | **C1D15 n = 28** | **C2D1 n =27** |
|---|---|---|---|
| T-cell | 105.9 / 59.1 | 182.0 / 90.7 | 116.4 / 62.9 |
| Monocyte | 18.1 / 10.0 | 5.3 / 2.6 | 4.0 / 2.5 |
| Granulocyte | 12.3 / 7.6 | 2.6 / 1.5 | 1.8 /1.3 |

| | | | |
|---|---|---|---|
| Markers for the cell types in Table 6 were characterized as outlined in Table 5. | | | |

Initial studies demonstrated that B-cell populations were depleted in the majority of patients due to prior exposure to anti-CD19 or anti-CD20 frontline NHL therapies. Quantification of H3K27me3 signal was therefore not possible in this population. T-cells exhibited the highest basal H3K27me3 levels followed by monocytes and granulocytes. Exposure to tazemetostat resulted in reductions of H3K27me3 in monocytes and granulocytes isolated from blood of all patients tested. The observed decrease was statistically significant for both cell populations at both post dose time points (T-test: p < 0.0001 in all cases). In contrast, the response of T-cells to tazemetostat treatment was a modest, though statistically significant, transient increase in H3K27me3 at C1D15 (p < 0.0001) followed by a return to baseline levels at C2D1. Some heterogeneity of T-cell H3K27me3 dynamics were observed across post dose time points from different patients.

Figure 12 illustrates a staining and gating scheme for flow cytometric analysis of peripheral mouse blood. Whole blood was collected in Na Heparin vacutainers followed by red blood cell lysis. Cells were fixed in 4% methanol-free formaldehyde and permeabilized with 0.1% Triton X-100. Processed cells were Fc-blocked and stained with anti-**CD3**-PerCP-Cy5.5, **CD45R**-BV421, **CD49b**-PE, **CD11b**-V500, **GR-1**-PE-Cy7, **H3**-AlexaFluor® 647 and **H3K27me3**-AlexaFluor® 488 antibodies.

Figure 13 illustrates the prevalence of the H3K27me3 methyl mark in mouse peripheral blood populations.The H3K27me3 levels are normalized to total H3 content for each population of interest. The pictured results demonstrate the rank order of H3K27me3/H3 in drug naive mouse peripheral blood leukocyte populations.

Figure 14 demonstrates differential sensitivity of mouse leukocyte populations to tazemetostat. The quantification of H3K27me3 / H3 in peripheral blood from a 7 day dose response study with EZH2 inhibitor tazemetostat presented in the graph reveals differential sensitivity of leukocyte populations: Monocyte and NK cells demonstrate high sensitivity to drug exposure; B-cells, T-cells and granulocytes exhibit low to moderate sensitivity. The results presented herein constitute a first description of a differential *in vivo* response to epigenetic inhibitor in a cell type specific manner.

Figure 15 shows cell type contribution to total H3K27me3 inhibition. Percent inhibition of normalized H3K27me3 is plotted against percent population composition in a mouse treated with 500mg/kg tazemetostat. The graph shows that highly sensitive monocyte and NK cell populations compose only 14% and 6% of total peripheral blood leukocytes. The total difference of H3K27me3/H3 levels at a dose of 500mg/kg based on contribution of individual populations was -18.1%.

Figure 16 compares H3K27me3 quantification from chromatin flow cytometry versus ELISA. The pictured results demonstrate good agreement with conventional ELISA quantification. The total inhibition by chromatin flow was calculated as the sum of fractional inhibition of all populations.

Figure 17 shows dose response to EPZ007210 in EZH2 mutant and wild type cell lines. H3K27me3 status is depicted on the flow cytometry plots following incubation with the following EPZ007210 concentrations DMSO only (yellow), 0.3nM (light blue), 1nM (magenta), 4nM (pink), 12nM (dark green), 37nM (light green), 111nM (orange), 333nM (teal) and 1000nM (red). The cell lines were treated with EPZ007210 for 4 days followed by fixation in 4% methanol free formaldehyde and permeabilization in 0.1% Triton X-100. Staining was performed in a single well using H3K27me3-AlexaFluor 488, H3K27me2-AlexaFluor-647 and H3-Pacific Blue. Mutant cell lines (WSU) exhibited tri-methylation but not di-methylation H3K27. Wild type lines (OCI-LY19) exhibited both di- and tri-methylation of H3K27. Furthermore, both cell lines demonstrated dose responsiveness of H3K27me3 with EPZ007210 treatment. The EZH2 wild type cell line also demonstrated a dose response of H3K27me2 with inhibitor treatment while no H3K27me2 was observed in the mutant line. Total H3 remained constant regardless of dosage or EZH2 mutational status. The experiments confirmed that chromatin flow cytometry is capable of detecting modulation of discrete H3K27 methylation states with inhibitor treatment and that it is possible to distinguish between multiple methylation states in a single assay.

Figure 18 illustrates a gating scheme for human peripheral blood following FACS. The following color scheme applies: Granulocytes are colored red, monocytes are colored blue, T cells are colored blue, and B-cells are colored magenta. Whole blood was collected in Na Heparin vacutainers followed by red blood cell lysis. Cells were fixed in 4% methanol-free formaldehyde and permeabilized with 0.1% Triton X-100. Processed cells were Fc-blocked and stained with anti-**CD3**-V500, **CD19**-BV421, **HLA-DR**-PE, **CD16**-PE-Cy7, **CD14-PerCP-Cy5.5, H3**-Alexa Fluor® 647, and **H3K27me3**-Alexa Fluor® 488.

Figure 19 shows the relative prevalence of the H3K27me3 methyl mark in human peripheral blood populations. The H3K27me3 levels are normalized to the total H3 content for each population of interest. The pictured results demonstrate the rank order of H3K27me3/H3 in drug naive human peripheral blood leukocyte populations, wherein the rank order demonstrates some inter-patient variability (e.g. B-cells have greater H3K27me3/H3 than T-cells in some individuals).

Figure 20 shows H3K27me3 inhibition in a NHL trial with tazemetostat treatment. Similar to what was found in rodent studies, monocytes were shown to be highly sensitive to inhibitor treatment. Granulocytes also demonstrated high sensitivity in humans. Furthemore, data not shown in the graph (N=1) suggests that a response is durable at day 30 of dosing. The figure constitutes a first demonstration of pharmacodynamic monitoring of epigenetic modulation by flow cytometry in human trials. Panel A) is a graph illustrating the change in total H3K27me3/H3 in peripheral blood of patients in NHL trial at cycle 1 day 1 and cycle 1 day 15. Panel B) is a graph showing the percent change of H3K27me3/H3 from baseline (cycle 1 day 1) to cycle 1 day 15.

Figure 21 shows Phase II pharmacodynamics for monocytes isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1). Monocytes exhibit robust inhibition of H3K27me3/H3 in response to tazemetostat exposure. Profiles of H3K27me3 inhibition in monocytes for patients with full longitudinal datasets to Cycle 2 Day 1 (day 45 exposure) demonstrate limited interpatient variability. Profiles of H3K27me3 inhibition vary significantly between patients. Specific profiles do not correlate with any clinical co-factors (e.g. age, sex, subtype).

Figure 22 shows Phase II pharmacodynamics for granulocytes isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1). Granulocytes exhibit robust inhibition of H3K27me3/H3 in response to tazemetostat exposure. Profiles of H3K27me3 inhibition in monocytes for patients with full longitudinal datasets to Cycle 2 Day 1 (day 45 exposure) demonstrate limited interpatient variability. Profiles of H3K27me3 inhibition vary significantly between patients. Specific profiles do not correlate with any clinical co-factors (e.g. age, sex, subtype).

Figure 23 shows Phase II pharmacodynamics for T-cells isolated by H3K27me3/H3 flow on blood collected from 51 patients dosed with 800 mg BID tazemetostat at cycle 2 day 1 (C2D1), cycle 2 day 15 (C1D15) and/or Cycle 2 day 1 (C2D1). T-cells demonstrate a modest, but statistically significant increase in H3K37me3 at day 15 (cycle 1 day 15) of tazemetostat exposure followed by a return to baseline at day 30 (cycle 2, day 1). Profiles of H3K27me3 inhibition in monocytes for patients with full longitudinal datasets to Cycle 2 Day 1 (day 45 exposure) demonstrate limited interpatient variability. Profiles of H3K27me3 inhibition vary significantly between patients. Specific profiles do not correlate with any clinical co-factors (e.g. age, sex, subtype). Changes in composition of T-cell subtypes (i.e. CD4, CD8, Treg) may influence observed profile of H3K27me3 modulation.

These results demonstrate that chromatin flow cytometry is an effective means to monitor H3K27me3 PD in PBMC subset isolated from DLBCL and FL patient blood treated with tazemetostat Different PMBC subsets demonstrate markedly divergent H3K27me3 PD profiles with monocytes and granulocytes exhibiting consistent and significant post dose reductions in H3K27me3 levels.

The details of one or more embodiments of the disclosure are set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

All patents and publications cited in this specification are incorporated by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow. Where names of cell lines or genes are used, abbreviations and names conform to the nomenclature of the American Type Culture Collection (ATCC) or the National Center for Biotechnology Information (NCBI), unless otherwise noted or evident from the context.

The invention disclosed herein can be embodied in other specific forms without departing from the spirit or essential characteristics thereof. The foregoing description has been presented only for the purposes of illustration and is not intended to limit the invention to the precise form disclosed, but by the claims appended hereto, and all changes that come within the meaning and range of equivalency of the claims are intended to be embraced therein.

The following statements represents different aspects of the invention. They are not claims:
1. A flow-cytometry method comprising
   quantifying trimethylation of lysine 27 of histone H3,
   quantifying total H3 level, and
   immunophenotyping of discrete cell populations.
2. The method of clause 1, wherein the discrete cell populations comprise a population of cells obtained from a subject.
3. A method, comprising, quantifying trimethylation of lysine 27 of Histone H3 in a population of cells obtained from a subject.
4. The method of clause 3, wherein the population of cells is isolated from peripheral blood or from a population of peripheral blood mononuclear cells obtained from the subject.
5. The method of clause 2 or 3, wherein the population of cells comprises, consists essentially, or consists of lymphoid cells.
6. The method of any one of clauses 2-4, wherein the population of cells comprises, consists essentially, or consists of myeloid cells.
7. The method of any one of clauses 2-6, wherein the population of cells comprises, consists essentially, or consists of myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells, or any combination thereof.
8. The method of any one of clauses 2-7, wherein the population of cells is characterized by expression of a cell surface antigen or a combination of cell surface antigens.
9. The method of clause 8, wherein the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof.
10. The method of clause 9, wherein the cell surface antigen or the combination thereof is CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR, or any combination thereof.
11. The method of any one of clauses 2-10, wherein the population of cells comprises, essentially consists of, or consists of myeloid stem or progenitor cells that are CD133⁺, CD271⁺, CD11⁺, and/or CD347⁺; erythroblasts that are CD71⁺; megakaryocytes that are CD61⁺; platelets that are CD61⁺; granulocytes that are HLA-DR⁺ and CD14⁻; basophils that are CD123⁺; eosinophils that are CD44⁺; neutrophils that are CD15⁺ and/or CD16⁺; monocytes that are CD14⁺, CD11b⁺, CD16⁻, and/or HLA-DR⁺; MDClc cells that are CD1c⁺; MDC2 cells that are CD141⁺; mast cells that are CD117⁺; lymphoid stem or progenitor cells that are CD34⁺, CD133⁺, CD271⁺, CD117⁺; T-lymphocytes (T-cells) that are CD2⁺ and/or CD3⁺; cytotoxic T cells that are CD8⁺; T helper cell that CD4⁺; regulatory T-cells that are CD4⁺ and CD25⁺; NK/T cell that are CD3⁺ and CD56⁺; natural killer T cells that are CD56⁺; activated T cells that are CD25⁺ and CD30⁺; B-cells that are CD19⁺ and/or CD20⁺; activated B-cells that are CD19⁺, CD25⁺, and/or CD30⁺; plasma cells that are CD138⁺; natural killer B cells that are CD56⁺; natural killer (NK) cells that are CD3⁻, CD19⁻, HLA-DR⁺ and CD16⁻; plasmacytoid dendritic cells that are CD304⁺; or any combination thereof.
12. The method of any one of clauses 2-11, wherein the population of cells comprises, essentially consists of, or consists of B-cells.
13. The method of any one of clauses 2-12, wherein the population of cells comprises, essentially consists of, or consists of T cells
14. The method of any one of clauses 2-13, wherein the population of cells comprises, essentially consists of, or consists of monocytes.
15. The method of clause 14, wherein the monocytes are CD14⁺ and CD16⁻; CD14^{low} and CD16⁺; or CD14^{high} and CD16^{low} monocytes, or any combination thereof.
16. The method of any one of clauses 2-15, wherein the population of cells comprises, essentially consists of, or consists of granulocytes.
17. The method of clause 16, wherein the granulocytes are neutrophils, eosinophils, or mast cells, or any combination thereof.
18. The method of any one of clauses 2-17, wherein quantifying trimethylation of lysine 27 of histone 3 comprises measuring a level of trimethylated lysine 27 in histone 3 in the population of cells, measuring the total level of histone 3 in the population of cells, and calculating a ratio of trimethylated lysine 27 level in histone 3 to the total histone 3 level in the population of cells.
19. The method of any one of clauses 2-18, wherein the method further comprises obtaining a blood sample or a sample of blood cells from the subject.
20. The method of any one of clauses 2-19, wherein the method further comprises isolating the population of cells.
21. The method of clause 20, wherein the isolating is by flow cytometry.
22. The method of clause 21, wherein the flow cytometry is based on the expression of one or more cell surface antigens.
23. The method of clause 22, wherein the isolating is by fluorescence-activated cell sorting (FACS).
24. The method of any one of clauses 2-23, wherein the subject
   (a) has been or is scheduled to be administered a therapeutically-effective amount of an EZH2 inhibitor, or
   (b) is identified as having increased H3K27me3 amounts in comparison to a control or reference value.
25. The method of any one of clauses 2-24, wherein the quantifying trimethylation of lysine 27 of histone H3 is performed before and after the subject is administered a therapeutically-effective amount of an EZH2 inhibitor.
26. The method of any one of clauses 2-25, wherein the method is used to monitor trimethylation of lysine 27 of histone H3 in a subject during a time period in which a therapeutically-effective amount of an EZH2 inhibitor is administered to the subject.
27. A method of quantifying an epigenetic modification of a histone in a sample from a subject, comprising:
   (a) contacting at least one cell in the sample with a permeabilizing composition to obtain a permeabilized sample;
   (b) contacting the permeabilized sample with a first fluorescent-conjugated antibody that specifically binds an epitope within H3K27me1, H3K27me2, or H3K27me3;
   (c) contacting the permeabilized sample with a second fluorescent-conjugated antibody that specifically binds an epitope within H3;
   (d) detecting a first fluorescent signal from the first fluorescent-conjugated antibody;
   (e) detecting a second fluorescent signal from the second-fluorescent conjugated antibody; and
   (d) determining an amount of H3 that is epigenetically-modified comprising dividing a value of the first fluorescent signal by a value of the second fluorescent signal,
      thereby quantifying the epigenetic modification of the histone in the sample.
28. The method of clause 27, further comprising sorting the at least one cell in the sample by cell size prior to contacting the permeabilized sample with either the first fluorescent-conjugated antibody or the second fluorescent-conjugated antibody.
29. The method of clause 27 or 28, further comprising immunophenotyping the at least one cell in the sample, comprising
   contacting the sample with a third fluorescent-conjugated antibody that specifically binds a third epitope on a cell type specific marker, and
   detecting a third fluorescent signal from the third fluorescent-conjugated antibody.
30. The method of clause 29, wherein the cell type specific marker is a cell surface antigen.
31. The method of clause 30, wherein the cell surface antigen is selected from the group consisting of CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR.
32. A method of monitoring a status of an epigenetic modification of a histone in a sample from a subject in need thereof comprising
   (a) determining a first quantity of an epigenetic modification of a histone according to the method of any one of claims 27-31 prior to an event;
   (b) determining a second quantity of the epigenetic modification of the histone according to the method of any one of claims 27-31 following the event; and
   (c) comparing the first quantity to the second quantity, wherein an increase indicates increased epigenetic modification following the event and a decrease indicates decreased epigenetic modification following the event.
33. The method of clause 32, wherein the event is a treatment comprising administration of an EZH2 inhibitor to the subject.
34. The method of clause 32 or 33, wherein the subject has or is diagnosed with cancer.
35. The method of any one of clauses 32-34, wherein the epigenetic modification is methylation of lysine 27 of histone 3.
36. A method for treating cancer in a subject in need thereof, comprising
   administering a therapeutically-effective amount of an EZH2 inhibitor to the subject, wherein the subject is identified as having increased H3K27me3 amounts in comparison to the amount of H3 according to the method of any one of clauses 27-31.
37. The method of any one of clauses 27-36, wherein the permeabilizing composition comprises a reagent selected from the group consisting of Triton X-100 (polyethylene glycol p(1,1,3,3-tetramethylbutyl)-phenyl ether), Nonidet P-40 (octylphenoxypolyethoxyethanol), Tween-20, saponin, digitonin, and n-octyl-β-D-glucopyranoside.
38. The method of clause 37, wherein the permeabilizing composition comprises Triton X-100 (polyethylene glycol p(1,1,3,3-tetramethylbutyl)-phenyl ether).
39. The method of clause 38, wherein the permeabilizing composition comprises between about 0.25% and about 5% wt/vol Triton X-100 (polyethylene glycol p(1,1,3,3-tetramethylbutyl)-phenyl ether).
40. The method of any one of clauses 27-39, wherein the first fluorescent signal is detected by flow cytometry.
41. The method of any one of clauses 27-40, wherein the second fluorescent signal is detected by flow cytometry.
42. The method of any one of clauses 29-41, wherein the third fluorescent signal is detected by flow cytometry.
43. The method of any one of the preceding clauses, wherein the sample comprises blood, skin, lymph fluid, lymph tissue, bone marrow, a hematological tumor, a liquid tumor, and a solid tumor.
44. The method of clause 43, wherein the blood sample comprises erythrocytes, lymphocytes, B cells, monocytes, eosinophils, basophils, neutrophils, thrombocytes, or a combination thereof.
45. The method of clause 44, wherein the sample comprises B cells.
46. The method of clause 43, wherein the lymph tissue and/or lymph fluid sample comprises endothelial cells, smooth muscle cells, pericytes, blood, lymph fluid or a combination thereof.
47. The method of clause 43, wherein the bone marrow sample comprises hematopoietic stem cells, stromal stem cells, myeloid tissue, yellow marrow, erythrocytes, leukocytes, or a combination thereof.
48. The method of any one of clauses 27-47, further comprising contacting the at least one cell in the sample with a fixing composition to obtain a fixed sample prior to contacting the fixed sample with the first fluorescently-conjugated antibody or the second fluorescently-conjugated antibody.
49. The method of clause 48, wherein the fixing composition is a methanol-free composition.
50. The method of clause 49, wherein the fixing composition is a methanol-free composition comprising formaldehyde at a concentration of between about 1% and about 10%.
51. The method of clause 50, wherein the methanol-free composition comprising formaldehyde is at a concentration of about 4%.
52. The method of clauses 33-51, wherein the EZH2 inhibitor is 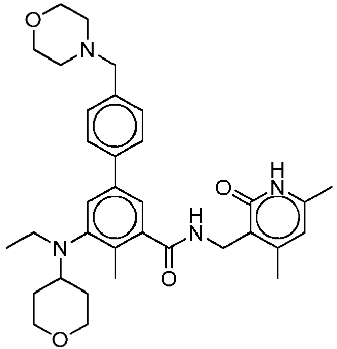 or a pharmaceutically acceptable salt thereof.
53. The method of any one of the preceding clauses, wherein the subject is a human.
54. The method of clause 53, wherein the human is 18 years old or younger.
55. The method of any one of the preceding clauses, wherein the subject has cancer.
56. The method of clause 55, wherein the cancer is a lymphoma.
57. The method of clause 56, wherein the lymphoma is selected from diffuse large B-cell lymphoma (DLBCL), a germinal center-derived lymphoma, a non-germinal center-derived lymphoma, follicular lymphoma (FL), primary mediastinal large B-cell lymphoma (PMBCL), marginal zone lymphoma (MZL), Burkitt's lymphoma and other non-Hodgkin's lymphoma subtype.

## Claims

1. A flow-cytometry method comprising
quantifying trimethylation of lysine 27 of histone H3,
quantifying total H3 level, and
immunophenotyping of discrete cell populations, preferably wherein the discrete cell populations comprise a population of cells obtained from a subject.

2. A method comprising quantifying trimethylation of lysine 27 of Histone H3 in a population of cells obtained from a subject, preferably wherein the population of cells is isolated from peripheral blood or from a population of peripheral blood mononuclear cells obtained from the subject.

3. The method of claim 1 or 2, wherein the population of cells:
(a) comprises, consists essentially, or consists of lymphoid cells;
(b) comprises, consists essentially, or consists of myeloid cells;
(c) comprises, consists essentially, or consists of myeloid stem or progenitor cells, erythroblasts, megakaryocytes, myeloblasts, platelets, granulocytes, basophils, eosinophils, neutrophils, promonocytes, monocytes, macrophages, myeloid dendritic cells, MDClc cells, MDC2 cells, mast cells, lymphoid stem or progenitor cells, thymocytes, T-lymphocytes, cytotoxic T cells, T helper cells, regulatory T-cells, natural killer T (NK/T) cells, activated T cells, B-cells, activated B-cells, plasma cells, natural killer B (NK/B) cells, natural killer (NK) cells, plasmacytoid dendritic cells, or any combination thereof;
(d) is **characterized by** expression of a cell surface antigen or a combination of cell surface antigens, preferably, wherein the cell surface antigen is CD1c, CD2, CD3, CD3, CD4, CD8, CD10, CD11, CD11b, CD14, CD15, CD16, CD19, CD20, CD24, CD25, CD28, CD30, CD34, CD38, CD40, CD44, CD45, CD45R, CD49b, CD56, CD61, CD71, CD95, CD117, CD123, CD133, CD138, CD141, CD150, CD184, CD271, CD347, GR-1, IgA, IgD, IgM, or HLA-DR, or any combination thereof, more preferably wherein the cell surface antigen or the combination thereof is CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR, or any combination thereof;
(e) comprises, essentially consists of, or consists of myeloid stem or progenitor cells that are CD133⁺, CD271⁺, CD11⁺, and/or CD347⁺; erythroblasts that are CD71⁺; megakaryocytes that are CD61⁺; platelets that are CD61⁺; granulocytes that are HLA-DR⁺ and CD14⁻; basophils that are CD123⁺; eosinophils that are CD44⁺; neutrophils that are CD15⁺ and/or CD16⁺; monocytes that are CD14⁺, CD11b⁺, CD16⁻, and/or HLA-DR⁺; MDClc cells that are CD1c⁺; MDC2 cells that are CD141⁺; mast cells that are CD117⁺; lymphoid stem or progenitor cells that are CD34⁺, CD133⁺, CD271⁺, CD117⁺; T-lymphocytes (T-cells) that are CD2⁺ and/or CD3⁺; cytotoxic T cells that are CD8⁺; T helper cell that CD4⁺; regulatory T-cells that are CD4⁺ and CD25⁺; NK/T cell that are CD3⁺ and CD56⁺; natural killer T cells that are CD56⁺; activated T cells that are CD25⁺ and CD30⁺; B-cells that are CD19⁺ and/or CD20⁺; activated B-cells that are CD19⁺, CD25⁺, and/or CD30⁺; plasma cells that are CD138⁺; natural killer B cells that are CD56⁺; natural killer (NK) cells that are CD3⁻, CD19⁻, HLA-DR⁺ and CD16⁻; plasmacytoid dendritic cells that are CD304⁺; or any combination thereof; and/or
(f) comprises, essentially consists of, or consists of (i) B-cells; (ii) T cells; (iii) monocytes, preferably wherein the monocytes are CD14⁺ and CD16⁻; CD14^{low} and CD16⁺; or CD14^{high} and CD16^{low} monocytes, or any combination thereof;or (iv) granulocytes, preferably wherein the granulocytes are neutrophils, eosinophils, or mast cells, or any combination thereof.

4. The method of any one of claims 1-3, wherein quantifying trimethylation of lysine 27 of histone 3 comprises measuring a level of trimethylated lysine 27 in histone 3 in the population of cells, measuring the total level of histone 3 in the population of cells, and calculating a ratio of trimethylated lysine 27 level in histone 3 to the total histone 3 level in the population of cells.

5. The method of any one of claims 2-4, wherein the method further comprises isolating the population of cells, preferably wherein the isolating is either by flow cytometry, preferably wherein the flow cytometry is based on the expression of one or more cell surface antigens; or by fluorescence-activated cell sorting (FACS).

6. The method of any one of claims 2-5, wherein:
(a) the subject:
(i) has been or is scheduled to be administered a therapeutically-effective amount of an EZH2 inhibitor, or
(ii) is identified as having increased H3K27me3 amounts in comparison to a control or reference value; and/or
(b) the quantifying trimethylation of lysine 27 of histone H3 is performed before and after the subject is administered a therapeutically-effective amount of an EZH2 inhibitor; and/or
(c) the method is used to monitor trimethylation of lysine 27 of histone H3 in a subject during a time period in which a therapeutically-effective amount of an EZH2 inhibitor is administered to the subject.

7. A method of quantifying an epigenetic modification of a histone in a sample from a subject, comprising:
(a) contacting at least one cell in the sample with a permeabilizing composition to obtain a permeabilized sample;
(b) contacting the permeabilized sample with a first fluorescent-conjugated antibody that specifically binds an epitope within H3K27me1, H3K27me2, or H3K27me3;
(c) contacting the permeabilized sample with a second fluorescent-conjugated antibody that specifically binds an epitope within H3;
(d) detecting a first fluorescent signal from the first fluorescent-conjugated antibody;
(e) detecting a second fluorescent signal from the second-fluorescent conjugated antibody; and
(f) determining an amount of H3 that is epigenetically-modified comprising dividing a value of the first fluorescent signal by a value of the second fluorescent signal,
thereby quantifying the epigenetic modification of the histone in the sample.

8. The method of claim 7, further comprising:
(a) sorting the at least one cell in the sample by cell size prior to contacting the permeabilized sample with either the first fluorescent-conjugated antibody or the second fluorescent-conjugated antibody; and/or
(b) immunophenotyping the at least one cell in the sample, comprising
contacting the sample with a third fluorescent-conjugated antibody that specifically binds a third epitope on a cell type specific marker, and
detecting a third fluorescent signal from the third fluorescent-conjugated antibody;
preferably wherein the cell type specific marker is a cell surface antigen, preferably wherein the cell surface antigen is selected from the group consisting of CD3, CD10, CD11b, CD14, CD16, CD19, CD20, CD24, CD28, CD34, CD38, CD40, CD45, CD45R, CD49b, CD95, CD150, CD184, GR-1, IgA, IgD, IgM, and HLA-DR.

9. A method of monitoring a status of an epigenetic modification of a histone in a sample from a subject in need thereof comprising:
(a) determining a first quantity of an epigenetic modification of a histone according to the method of claim 7 or claim 8 prior to an event;
(b) determining a second quantity of the epigenetic modification of the histone according to the method of claim 7 or claim 8 following the event; and
(c) comparing the first quantity to the second quantity, wherein an increase indicates increased epigenetic modification following the event and a decrease indicates decreased epigenetic modification following the event, preferably wherein:
(i) the event is a treatment comprising administration of an EZH2 inhibitor to the subject;and/or
(ii) the subject has or is diagnosed with cancer; and/or
(iii) the epigenetic modification is --methylation of lysine 27 of histone 3.

10. A method for treating cancer in a subject in need thereof, comprising administering a therapeutically effective amount of an EZH2 inhibitor to the subject, wherein the subject is identified as having increased H3K27me3 amounts in comparison to the amount of H3 according to the method of any one of claim 7-9.

11. The method of any one of claims 7-10, wherein the permeabilizing composition comprises a reagent selected from the group consisting of Triton X-100 (polyethylene glycol p(1,1,3,3-tetramethylbutyl)-phenyl ether), Nonidet P-40 (octylphenoxypolyethoxyethanol), Tween-20, saponin, digitonin, and n-octyl-β-D-glucopyranoside; preferably wherein the permeabilizing composition comprises Triton X-100 (polyethylene glycol p(1,1,3,3-tetramethylbutyl)-phenyl ether),
preferably wherein the permeabilizing composition comprises between about 0.25% and about 5% wt/vol Triton X-100 (polyethylene glycol p(1,1,3,3-tetramethylbutyl)-phenyl ether).

12. The method of any one of claims 7-11, wherein:
(a) the first fluorescent signal is detected by flow cytometry; and/or
(b) the second fluorescent signal is detected by flow cytometry; and/or
(c) the third fluorescent signal is detected by flow cytometry.

13. The method of any one of the preceding claims, wherein:
(a) the sample comprises blood, skin, lymph fluid, lymph tissue, bone marrow, a hematological tumor, a liquid tumor, and a solid tumor, preferably wherein:
(i) the blood sample comprises erythrocytes, lymphocytes, B cells, monocytes, eosinophils, basophils, neutrophils, thrombocytes, or a combination thereof;
(ii) the lymph tissue and/or lymph fluid sample comprises endothelial cells, smooth muscle cells, pericytes, blood, lymph fluid or a combination thereof; and/or
(iii) the bone marrow sample comprises hematopoietic stem cells, stromal stem cells, myeloid tissue, yellow marrow, erythrocytes, leukocytes, or a combination thereof; and/or
(b) the subject is:
(i) a human, preferably wherein the human is 18 years old or younger; and/or
(ii) the subject has cancer; preferably wherein the cancer is a lymphoma, preferably wherein the lymphoma is selected from diffuse large B-cell lymphoma (DLBCL), a germinal center-derived lymphoma, a non-germinal center-derived lymphoma, follicular lymphoma (FL), primary mediastinal large B-cell lymphoma (PMBCL), marginal zone lymphoma (MZL), Burkitt's lymphoma and other non-Hodgkin's lymphoma subtype.

14. The method of any one of claims 7-13, further comprising contacting the at least one cell in the sample with a fixing composition to obtain a fixed sample prior to contacting the fixed sample with the first fluorescently-conjugated antibody or the second fluorescently-conjugated antibody, preferably wherein the fixing composition is a methanol-free composition,
preferably wherein the methanol-free composition comprises: formaldehyde at a concentration of between about 1% and about 10%; or formaldehyde at a concentration of about 4%.

15. The method of claim 11-15, wherein the EZH2 inhibitor is 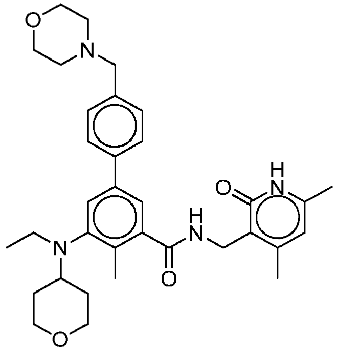 or a pharmaceutically acceptable salt thereof.
